# EUROPEAN PATENT APPLICATION

(11) **EP 4 785 944 A1**
(43) Date of publication of application: **05.08.2026**
(21) Application number: 24872402.3
(22) Date of filing: 26.09.2024
(51) Int. Cl.: A61K 31/506, A61K 9/20, A61K 9/30, A61K 47/14, A61K 47/26, A61K 47/32, A61K 47/36, A61K 47/38, A61P 35/00, A61P 35/02

(54) **PREPARATION EXCELLENT IN ELUTION PROPERTY AND TABLETING PROPERTY**

(30) Priority: 29.09.2023 JP 2023169918
(71) Applicant: Sumitomo Pharma Co., Ltd., Osaka-shi, Osaka 541-0045 (JP)
(72) Inventor: YOSHINO, Saori, Osaka-shi, Osaka 541-0045 (JP); IKARI, Hiroki, Suita-shi, Osaka 564-0053 (JP); ITO, Koichiro, Suita-shi, Osaka 564-0053 (JP); ASADA, Takumi, Suita-shi, Osaka 564-0053 (JP); ICHIBAYASHI, Eri, Suita-shi, Osaka 564-0053 (JP)
(74) Representative: Vossius & Partner Patentanwälte Rechtsanwälte mbB
(86) International application number: PCT/JP2024/034441
(87) International publication number: WO 2025/070617

(57) **Abstract**

The present invention relates to an oral solid formulation comprising 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide as an active ingredient, which has good dissolution properties and storage stability.

## Description

### TECHNICAL FIELD

The present invention relates to an oral solid formulation comprising 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide (hereinafter, it may be referred to as "the present compound") or a pharmaceutically acceptable salt thereof, or a hydrate or solvate thereof (hereinafter, all the above may be referred to as "medicament", "the present medicament", or "the medicament used herein") as an active ingredient, which has good dissolution properties and storage stability, and further, when manufactured as a tablet, suppresses tableting troubles.

### BACKGROUND OF THE INVENTION

It is important to obtain dose-response information in clinical development programs. According to Non-Patent Literature 1, dose-response information is obtained by collecting necessary information at each stage throughout non-clinical and clinical development, and the purpose of obtaining the information is to utilize the useful information in subsequent clinical trials and post-marketing use. In other words, it is important to understand the relationship between dose, blood concentration, and clinical response (efficacy and safety), to identify the appropriate starting dose for a group or individual patient, the best way to adjust the dose to suit the needs of a specific patient, and the dose at which no further benefit is expected even if increased, or at which an increase is likely to cause unacceptable side effects, and to utilize these in the next stage. Furthermore, since the dose may be changed during clinical development, it is preferable to obtain oral solid formulations that cover a wide range of doses, from low to high, in order to obtain such information.

When a solid formulation is orally administered, in order to exert its effects, the dissolved drug must reach the absorption site after going through the processes of disintegration, dispersion, and dissolution. Since this process has a significant influence on drug absorption, it is desirable to design the formulation so that it can maintain stable and good dissolution in order to exert and maintain its pharmacological effect.

Patent Literature 1 discloses a formulation of the present compound, but does not disclose any formulation examples showing specific ingredients, compositions, or manufacturing methods.

### PRIOR ART

### [Patent Reference]

[Patent Literature 1] WO2020/045334

### [Non-patent Reference]

[Non-Patent Literature 1] Dose-Response Information to Support Drug Registration (ICH E4)

### SUMMARY OF THE INVENTION

### (Technical Problem)

The purpose of the present invention is to provide an oral solid formulation comprising the present medicament, which has good dissolution properties and storage stability, and further, when manufactured as a tablet, suppresses tableting troubles.

### (Solution to Problem)

When the present medicament is made into an oral solid formulation, it has become clear that tableting troubles occur during the tablet production process (*i.e.,* the tableting process), such as the phenomenon of the composition comprising the present medicament adhering to the surface of the punch (so-called sticking) and the phenomenon of the surface peeling off (so-called capping). In general, the addition of a binder and a lubricant tends to improve tableting troubles during the tableting process. However, it is also known that increasing the amount of binder and lubricant added beyond an appropriate amount can delay the dissolution rate of the compound and reduce the tablet moldability (reduction in hardness). However, the present inventors have extensively studied, and then have surprisingly found that by using a binder and a lubricant in amounts greater than the usual composition ratios, troubles such as delayed dissolution and reduced hardness were not observed, and the fluidity can be improved and the tableting trouble can be resolved.

The present inventors have extensively studied, and then have found that it is possible to provide an oral solid formulation having good dissolution properties and storage stability by adding higher amounts of both a certain lubricant and a certain binder than are normally used. Specifically, the present inventors have found that even in tablets comprising a high content of hydroxypropyl cellulose as a binder and sodium stearyl fumarate as a lubricant, there is no delayed dissolution or reduction in hardness, tableting troubles are suppressed, and good dissolution properties are maintained while good storage stability is obtained. Based upon the findings, the present invention has been achieved.

The present invention provides the following items.

### (Item 1)

### An oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 4.0 - 6.0% (w/w) per 100% (w/w) of the formulation, and
(3) a lubricant in 2.0 - 10.0% (w/w) per 100% (w/w) of the formulation.

### (Item 2)

The oral solid formulation of Item 1, wherein the content of the binder is 5.0 - 6.0% (w/w) per 100% (w/w) of the formulation.

### (Item 3)

The oral solid formulation of Item 1 or 2, wherein the binder is a water-soluble polymer binder.

### (Item 4)

The oral solid formulation of Item 3, wherein the water-soluble polymer binder is hydroxypropyl cellulose or polyvinyl alcohol.

### (Item 5)

The oral solid formulation of Item 3, wherein the water-soluble polymer binder is hydroxypropyl cellulose.

### (Item 6)

The oral solid formulation of any one of Items 1 to 5, wherein the content of the lubricant is 2.0 - 6.0% (w/w) per 100% (w/w) of the formulation.

### (Item 7)

The oral solid formulation of any one of Items 1 to 5, wherein the content of the lubricant is 4.0 - 6.0% (w/w) per 100% (w/w) of the formulation.

### (Item 8)

The oral solid formulation of any one of Items 1 to 7, wherein the lubricant is sodium stearyl fumarate.

### (Item 9)

The oral solid formulation of any one of Items 1 to 8, which further comprises an excipient.

### (Item 10)

The oral solid formulation of Item 9, wherein the excipient is a hydrophilic excipient.

### (Item 11)

The oral solid formulation of Item 10, wherein the hydrophilic excipient is any one selected from sugar alcohol, pregelatinized starch, lactose hydrate, and microcrystalline cellulose, or a combination of two or more thereof.

### (Item 12)

The oral solid formulation of Item 10, wherein the hydrophilic excipient is any one selected from sugar alcohol, pregelatinized starch, and microcrystalline cellulose, or a combination of two or more thereof.

### (Item 13)

The oral solid formulation of Item 10, wherein the hydrophilic excipient is any one selected from sugar alcohol and pregelatinized starch, or a combination of two or more thereof.

### (Item 14)

The oral solid formulation of Item 10, wherein the hydrophilic excipient is a combination of sugar alcohol and pregelatinized starch.

### (Item 15)

The oral solid formulation of any one of Items 11 to 14, wherein the pregelatinized starch is partially pregelatinized starch.

### (Item 16)

The oral solid formulation of any one of Items 11 to 15, wherein the sugar alcohol is D-mannitol.

### (Item 17)

The oral solid formulation of any one of Items 1 to 16, which further comprises a disintegrant.

### (Item 18)

The oral solid formulation of Item 17, wherein the content of the disintegrant is 0.5 - 50.0% (w/w) per 100% (w/w) of the formulation.

### (Item 19)

The oral solid formulation of Item 17, wherein the content of the disintegrant is 0.5 - 30.0% (w/w) per 100% (w/w) of the formulation.

### (Item 20)

The oral solid formulation of Item 17, wherein the content of the disintegrant is 0.5 - 20.0% (w/w) per 100% (w/w) of the formulation.

### (Item 21)

The oral solid formulation of any one of Items 17 to 20, wherein the disintegrant is any one selected from croscarmellose sodium, sodium starch glycolate, and crospovidone, or a combination of two or more thereof.

### (Item 22)

The oral solid formulation of any one of Items 17 to 20, wherein the disintegrant is any one selected from croscarmellose sodium and sodium starch glycolate, or a combination of two or more thereof.

### (Item 23)

The oral solid formulation of any one of Items 17 to 20, wherein the disintegrant is croscarmellose sodium.

### (Item 24)

The oral solid formulation of any one of Items 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof is 0.5 - 70% (w/w) per 100% (w/w) of the formulation.

### (Item 25)

The oral solid formulation of any one of Items 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof is 10 - 50% (w/w) per 100% (w/w) of the formulation.

### (Item 26)

The oral solid formulation of any one of Items 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof is 15 - 40% (w/w) per 100% (w/w) of the formulation.

### (Item 27)

The oral solid formulation of any one of Items 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof is 15 - 38% (w/w) per 100% (w/w) of the formulation.

### (Item 28)

The oral solid formulation of any one of Items 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof is 20 - 38% (w/w) per 100% (w/w) of the formulation.

### (Item 29)

The oral solid formulation of any one of Items 1 to 28, which is prepared by fluid-bed granulation.

### (Item 30)

The oral solid formulation of any one of Items 1 to 29, which is film-coated with a coating agent.

### (Item 31)

The oral solid formulation of any one of Items 1 to 30, which is in use for treating and/or preventing a tumor.

### (Effect of the Invention)

The oral solid formulation of the present invention comprises a binder and a lubricant in amounts higher than those usually used, making it possible to provide an oral solid formulation with good dissolution properties and storage stability. Furthermore, by using a high content of binder and lubricant, it is possible to suppress tableting troubles, maintain good dissolution properties, and provide tablets with good storage stability.

### DETAILED DESCRIPTION OF THE PREFERRED EMBODIMENTS

The details of the present invention are explained as follows. The embodiments provided below are provided for a better understanding of the present invention, and the scope of the present invention is not limited to the following description. And, the following embodiments can be used alone or in combination.

The term "average particle size" used herein refers to the cumulative 50% particle size D50 measured on a volume basis of powder particles. The mean particle size is measured on a volume basis using a laser diffraction particle size distribution measuring device (for example, Particle Viewer manufactured by Powrex Corporation, SALD-3000J manufactured by Shimadzu Corporation, or HELOS & RODOS manufactured by Sympatec).

"Tableting trouble" used herein refers to the phenomenon in which the present compound adheres to the surface of the punch during the tablet production process (*i.e.,* the tableting process) (sticking), the phenomenon in which the surface peels off (capping), and the phenomenon in which friction occurs between the powder being tableted and the metal adhesion surface, causing scratches on the side of the tablet (die friction).

In the present invention, unless otherwise specified, the term "content" refers to the adding amount or content (wt/wt) when the total amount of the formulation is taken as 100% (w/w). "% (w/w)" is also represented as "wt %".

In the present invention, "good storage stability" refers to a formulation characteristic in which the increase in the amount of related substances is small in a purity test of a storage stability test, and good dissolution properties are maintained in a dissolution test after storage.

"Storage" refers to leaving, storing, or keeping a manufactured formulation in an appropriate container. During storage, the container may be sealed or open, and may or may not be protected from light. Examples of storage conditions include storage at ordinary temperature, at room temperature, in a cool place, under refrigeration, or under freezing. Storage temperatures include, but are not limited to, standard temperature: 20°C, ordinary temperature: 15-25°C, room temperature: 1 to 30°C, lukewarm temperature: 30 to 40°C, cool place: 1 to 15°C, refrigeration: 2 to 6°C, and freezing: approximately -20 to -18°C. The relative humidity (% RH) during storage may be anywhere from 0 to 100% RH, and is preferably 40 to 60% RH, but is not limited to these. Storage periods include, but are not limited to, hours, days, weeks, months, and years. The storage parameters can be appropriately selected depending on the properties of the formulation and storage conditions. "Storage stability testing" is a stability testing to confirm whether the quality of the manufactured formulation is maintained, and is a testing conducted under storage conditions that promote chemical or physical changes in the formulation. The test results can be used to evaluate the chemical influences of long-term storage using the specified storage methods. It can also be used to assess the impact of short-term deviations from storage methods, that may occur during transport. Depending on the temperature/relative humidity, long-term tests (e.g., 25°C/60% RH), accelerated tests (e.g., 40°C/75% RH), and severe tests (e.g., 50°C/85% RH) can be selected. The accelerated test or stress test used herein is carried out by setting the conditions of a thermo-hygrostat to a higher temperature/higher relative humidity (e.g., 40°C/75% RH or 50°C/85% RH, respectively), placing the prepared solid formulation in an appropriate container (e.g., an HDPE bottle (material: high density polyethylene (HDPE)) or an amber screw-cap test tube (material: glass)), and storing the container in an opened or closed state in the thermo-hygrostat for a certain storage period (e.g., 1, 3, or 6 months, or 2 or 4 weeks, in each condition). In order to provide patients with high-quality formulations, it is necessary to keep the amount of related substances below a specified level in storage stability tests. As a general criterion, the total amount of related substances is not more than 3.0%.

The term "good dissolution properties" used herein means that, for example, when a dissolution test of the formulation is performed in accordance with the Paddle Method of the Dissolution Test of the 18th Edition of the Japanese Pharmacopoeia under the test conditions described below, the formulation exhibits a dissolution rate of 80% or more, preferably 85% or more, at 15 minutes.

Another aspect of "good dissolution properties" is that the dissolution rate is 70% or more, preferably 80% or more, and more preferably 85% or more at 10 minutes. Another aspect of "good dissolution properties " is that the dissolution rate is 80% or more, and preferably 85% or more, at 5 minutes.
Test solution: 0.1 mol/L hydrochloric acid test solution
Paddle rotation speed: 50 rpm
Test medium: 900 mL
Medium temperature: 37±0.5°C

### (1) Oral solid formulation

The term "oral solid preparation" refers to a solid formulation of a certain shape, that is administered orally. Solid formulations include those formulated into dosage forms such as tablets, capsules, granules, fine granules, pills, and powders.

The oral solid formulation used herein particularly refers to those formulated into tablets, capsules, granules, and fine granules. Preferred examples of the oral solid formulation include tablets and capsules. A more preferred example of the oral solid formulation is tablets.

The oral solid formulation used herein (for example, tablet) may be film-coated with a coating agent for the purposes of facilitating administration or preventing decomposition of the active ingredient. In this case, the oral solid formulation of the present disclosure can be said to be a film-coated tablet. Film-coated tablets are usually formulated by film-coating an uncoated tablet with an appropriate coating agent such as a polymer compound.

The oral solid formulation used herein comprises (1) a medicament, (2) a binder, and (3) a lubricant, and may further comprise, as necessary, (4) an excipient, (5) a disintegrant, and optionally other (6) additives to the extent that they do not affect the properties of the formulation of the present invention.

In the case of tablets, the form thereof includes uncoated tablets, film-coated tablets in which a film coating is applied to the surface of an uncoated tablet (sometimes referred to as FC tablets in the present specification), and sugar-coated tablets in which a sugar coating is applied to the surface of an uncoated tablet, and uncoated tablets or film-coated tablets are preferred.

### (1) Medicament

The term "medicament" used herein refers to the active ingredient of the present invention, 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide (*i.e.*, the present compound) or a pharmaceutically acceptable salt thereof, or a hydrate or solvate thereof, which has the following structure. 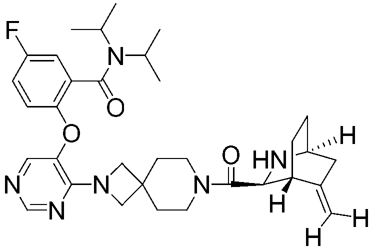 In addition, the term "a hydrate or solvate thereof" in "the present compound or a pharmaceutically acceptable salt thereof, or a hydrate or solvate thereof" refers to a hydrate or solvate of the present compound, and a hydrate or solvate of a pharmaceutically acceptable salt of the present compound.

The "pharmaceutically acceptable salt" used herein includes, but not limited to, hydrochloride, citrate, fumarate, maleate, phosphate, succinate, sulfate, tartrate, besylate, and hydrobromide.

The term "tartrate of the present compound" refers to 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide 1L (+)-tartrate.

The term "hydrate" used herein refers to a solid state in which the molecule of the present compound contains a certain ratio of water molecules; and the term "solvate" refers to a state in which the water molecules are replaced by organic solvent molecules. Organic solvents capable of forming the present solvate can be appropriately selected depending on the type and character of the substance, which include, for example, alcohols such as ethanol and propanol, acetone and ethyl acetate. The number of solvent molecules in hydrate or solvate can be appropriately selected depending on the stability of the hydrate/solvate, etc.

The content of the medicament in the present invention is generally 0.1 - 96% (w/w), preferably 0.5 - 70% (w/w), more preferably 5 - 60% (w/w), 5 - 50% (w/w), 10 - 60% (w/w), 10 - 50% (w/w), 15 - 60% (w/w), 15 - 50% (w/w), 10 - 40% (w/w), 15 - 40% (w/w), 20 - 40% (w/w), 20 - 38% (w/w), 15 - 38% (w/w), 15 - 36% (w/w), or 20 - 36% (w/w), and further preferably 20 - 30% (w/w), per 100% (w/w) of the formulation.

The medicament may be micronized to a desired particle size as necessary during formulation. The mean particle size of the medicament is generally 0.1 - 100 µm, preferably 0.1 - 80 µm, more preferably 0.1 - 50 µm, and even more preferably 1 - 20 µm. The mean particle size of the medicament as a drug substance may be within the above range, and may vary during the manufacturing process, etc.

### (2) Binder

"Binder" is an ingredient that is used to give binding strength to powder and to create and maintain the shape of the formulation, and a preferred binder in the present invention is a water-soluble polymer binder. Examples of "water-soluble polymer binder" includes, but is not limited to, methylcellulose, hydroxypropyl cellulose, hydroxypropyl methylcellulose, hydroxyethyl cellulose, hydroxymethyl cellulose, carboxymethyl cellulose, polyvinylpyrrolidone, polyvinyl alcohol, copolyvidone, polyethylene glycol, polyvinyl alcohol-acrylic acid-methyl methacrylate copolymer, vinyl acetate-vinylpyrrolidone copolymer, polyvinyl alcohol-polyethylene glycol graft copolymer, pregelatinized starches, dextrin, dextran, pullulan, alginic acid, gelatin, and pectin; preferably hydroxypropyl cellulose, hydroxypropyl methylcellulose, and polyvinyl alcohol; further preferably hydroxypropyl cellulose. The above-listed water-soluble polymer binders can be used alone or in combination of two or more. Hydroxypropyl cellulose which is a typical binder is generally used at concentration of 2% - 3%.

The use of hydroxypropyl cellulose as a water-soluble polymer binder allows for successful preparation of drug-containing granules (*i.e.,* granulation) in formulation, and also allows for the production of formulations that are easy to handle and have good disintegration properties.

The viscosity of hydroxypropyl cellulose is not particularly limited, but when made into a 2% aqueous solution at 20°C, it has a viscosity of 2 to 400 mPa·s, and preferably 2 to 10 mPa·s.

The content of the binder is generally 4.0 - 20% (w/w), preferably 4.0 - 10% (w/w), more preferably 4.0 - 6.0% (w/w), 4.5 - 6.0% (w/w), 5.0 - 6.0% (w/w), 5.5 - 6.0% (w/w), 4.0 - 5.5% (w/w), 4.5 - 5.5% (w/w), 5.0 - 5.5% (w/w), 4.0 - 5.0% (w/w), or 4.5 - 5.0% (w/w), and further preferably 5.0 - 5.5% (w/w) or 5.0 - 6.0% (w/w), per 100% (w/w) of the formulation.

The binder of the present invention can be dissolved in a solvent such as water, and the granulation can be done while spraying the binder solution. In addition, the binder can be mixed with other ingredients, and the granulation can be also done while spraying a solvent such as water.

### (3) Lubricant

"Lubricant" is an ingredient that is added during the manufacturing process of capsules and tablets to improve powder fluidity, improve filling properties, and prevent adhesion when filling capsules or compressing tablets. In the case of tablets, lubricant may be mixed with other ingredients before tableting, or may be sprayed onto punches and dies during tableting.

The lubricant used herein is not particularly limited, but it includes, for example, magnesium stearate, sodium stearyl fumarate, talc, polyethylene glycol, silica, and hydrogenated vegetable oil; more preferably magnesium stearate and sodium stearyl fumarate, and even more preferably sodium stearyl fumarate. The above-listed lubricants can be used alone or in combination of two or more. When sodium stearyl fumarate is used as a lubricant, tableting trouble during tableting can be suppressed to prepare a formulation with excellent handleability and good disintegrability. Lubricants are generally contained in amounts of 1% or less, and at most up to about 2%, and a commonly used lubricant is magnesium stearate.

The content of the lubricant is generally 0.1 - 20% (w/w), preferably 2.0 - 10% (w/w), more preferably 4.0 - 10% (w/w), 5.0 - 10% (w/w), 2.0 - 6.0% (w/w), 2.5 - 6.0% (w/w), 3.0 - 6.0% (w/w), 3.5 - 6.0% (w/w), 4.5 - 6.0% (w/w), or 5.0 - 6.0% (w/w), and further preferably 4.0 - 6.0% (w/w), per 100% (w/w) of the formulation.

### (4) Excipient

"Excipient" is an ingredient that is added to create a dosage form, increase the volume, or dilute the formulation material to make it easier to handle when an active ingredient alone does not provide sufficient "bulk" to give the formulation a certain shape. In addition to simply increasing the volume, excipients also have functions such as improving the mixability of powders, improving the granulation properties when preparing particles in granules, improving the filling properties, adhesion, and fluidity into a die when tableting, and improving the filling properties of capsules.

The "excipient" used herein includes hydrophilic excipients and non-hydrophilic excipients, preferably hydrophilic excipients.

As such hydrophilic excipient, any hydrophilic excipients that are commonly used in formulation can be used, and preferred examples include sugar, microcrystalline cellulose, and sugar alcohol.

The type of sugar or sugar alcohol used herein is not particularly limited, but it includes, for example, pregelatinized starches, D-mannitol, erythritol, xylitol, maltitol, sorbitol, lactose, sucrose, and toruhalose; preferably D-mannitol, erythritol, lactose, and toruhalose; more preferably pregelatinized starches, D-mannitol, and lactose; and most preferably pregelatinized starches and D-mannitol. Such hydrophilic excipient may be one selected from the above or a combination of two or more thereof.

The content of the excipient is generally 0.1 - 90% (w/w), preferably 10 - 90% (w/w), more preferably 30 - 80% (w/w), and further preferably 40 - 70% (w/w), per 100% (w/w) of the formulation.

The content of D-mannitol is generally 0.1 - 90% (w/w), preferably 1 - 80% (w/w), and more preferably 30 - 60% (w/w), per 100% (w/w) of the formulation.

"Pregelatinized starches" includes, for example, pregelatinized starch and partially pregelatinized starch in the Pharmaceutical Excipients Standards, as well as "pregelatinized starch" in USP/NF and "starch, pregelatinized" in Ph. Eur. A preferred example of the "pregelatinized starches" is "partially pregelatinized starch." Specific examples of commercially available pregelatinized starch or partially pregelatinized starch include PC-10 (trade name, sold by Asahi Kasei Corporation), SWELSTAR (trade name, sold by Asahi Kasei Corporation), Starch 1500 and Starch 1500G (trade name, sold by Colorcon), and LYCATAB C (trade name, sold by Roquette), but are not limited thereto. Any pregelatinized starch or partially pregelatinized starch may be used as long as its ingredient is pregelatinized starch or partially pregelatinized starch and its purity and other specifications satisfy the standards of a person skilled in the art. Examples of raw materials for pregelatinized starches include various starches such as corn starch, potato starch, wheat starch, rice starch, and tapioca starch.

The content of such pregelatinized starches is generally 0.1 to 50% (w/w), preferably 1 - 40% (w/w), and more preferably 10 - 30% (w/w), per 100% (w/w) of the formulation.

### (5) Disintegrant

"Disintegrant" is an ingredient that is added for the purpose of disintegrating and dispersing solid formulations such as tablets or granules into particles.

The "disintegrant" used herein is not particularly limited, but it includes, for example, cornstarch, croscarmellose sodium, sodium starch glycolate (or sodium carboxymethyl starch), low-substituted hydroxypropyl cellulose, carmellose, carmellose calcium, carboxymethylethyl cellulose, and crospovidone, preferably croscarmellose sodium, sodium starch glycolate, low-substituted hydroxypropyl cellulose, carmellose, carmellose calcium, and crospovidone, more preferably croscarmellose sodium, sodium starch glycolate, and crospovidone, and further preferably croscarmellose sodium. Such disintegrant may be one selected from the above or a combination of two or more thereof.

The content of the disintegrant is generally 0.1 - 50.0% (w/w), preferably 0.5 - 50.0% (w/w), 0.5 - 30.0% (w/w), more preferably 0.5 - 20% (w/w), 0.5 - 30.0% (w/w), 1.0 - 30.0% (w/w), or 1.0 - 20.0% (w/w), and further preferably 0.5 - 20.0% (w/w), 2.0 - 10.0% (w/w), 1.0 - 10.0% (w/w), or 2.0 - 5.0% (w/w).

### (6) Additive

"Additives" refers to ingredients other than active ingredients contained in a formulation, that are used for purposes such as enhancing the usefulness of the active ingredients and formulations, facilitating formulation, stabilizing quality, and improving usability.

In the present formulation disclosed herein, non-toxic and inactive additives commonly used in the pharmaceutical field can be added to the formulation, as appropriate, as long as they do not affect the action of the medicament of the present disclosure. The additives used herein include those that do not affect the therapeutic effect of the medicament disclosed herein and are used in general oral formulations. Additives include, but are not limited to, for example, stabilizers, flavoring agents, sweeteners, odorants, fragrances, antioxidants, antistatic agents, fluidizing agents, coloring agents, plasticizers, anti-agglomerating agents, and glossing agents.

The content of the additives can be set arbitrarily, but it is, for example, 0.001 - 10% (w/w), preferably 0.005 - 10% (w/w), and more preferably 0.01 - 5% (w/w), per 100% (w/w) of the formulation. Another preferred embodiment is a formulation that does not comprise any additives.

"Coating agent" is an ingredient used to cover the surface of a formulation, which is used to prevent contact with water, air, or light, to mask odors or bitterness, to impart formulation properties such as sustained release and enteric properties, or to improve the appearance and increase commercial value.

The coating agent used herein is not particularly limited, but it includes, for example, a combination of a base material such as hypromellose, hydroxypropyl cellulose, polyvinylpyrrolidone, polyvinyl alcohol, polyvinyl alcohol-polyethylene glycol-graft copolymer, methacrylic acid copolymer L, methacrylic acid copolymer LD, methacrylic acid copolymer S, aminoalkyl methacrylate copolymer RS, and ethyl acrylate-methyl methacrylate copolymer; and a plasticizer such as polyethylene glycol, propylene glycol, triacetin, triethyl citrate, glycerin, and glycerin fatty acid esters. And, additives such as titanium oxide, iron oxide, talc, colorants, etc. may also be added to the coating agent. And, mixtures with these additives may also be used, such as OPADRY.

"Coloring agent" is an ingredient used to identify capsules, tablets, etc., to shield the contained medicament from light, or to increase commercial value. The "coloring agent" used herein is not particularly limited, but it includes, for example, coal-tar dyes, lake pigments, yellow ferric oxide, red ferric oxide, and titanium oxide. Further, after film coating, carnauba wax, talc, etc. may be added as a glossing agent.

The preparation of the present oral formulation varies depending on the desired dosage form, but the desired dosage form can be prepared according to conventional methods.

### (1) Preparation of aqueous solution comprising binder:

A binder is dissolved in purified water. The amount of binder is selected, for example, from the range of 1 to 20% (w/w), preferably from the range of 2 to 8% (w/w), relative to the amount of purified water.

### (2) Preparation of granulated product comprising the present medicament:

The present medicament, a water-soluble excipient, and a disintegrant are charged into a granulator, and the granulation is carried out while the binder solution prepared in step (1) above is sprayed on the mixture. Or, the granulation can also be carried out by spraying a solvent such as water into a granulator containing the present medicament, a water-soluble excipient, a disintegrant, and a binder.

Examples of granulator include, but are not limited to, a granulation equipment classified into fluid bed granulation, high-share granulation, roto fluid bed granulation, or twin screw granulation.

### (3) Drying of granulated product:

The above granulated product is dried under reduced pressure or normal pressure. The drying is carried out so that the loss on drying measured with an infrared moisture meter is, for example, 4% (w/w) or less, preferably 2% (w/w) or less.

### (4) Addition of lubricant:

To the granules dried in step (3) is added a lubricant, and the mixture is mixed. For the mixing, for example, a mixer classified as a diffusion mixer (tumble) is used. It includes, but is not limited to, for example, tumble blender, V blender, double cone, and bin tumbler.

### (5) Tablet compression:

The above mixture is compressed to prepare tablets. As a tableting device, for example, a tableting machine classified as a tablet press is used. The tableting pressure is set so that the tablet hardness falls within the range of, for example, 20 to 250 N.

### (6) Applying film coating if desired:

The above-prepared tablets may be film-coated as needed. As a coating device, for example, a device classified as a coating pan is used. A preferred example includes a device classified as a perforated coating system.

### (7) Drying:

The above-prepared tablet is dried. The drying is carried out under reduced pressure or normal pressure so that the loss on drying measured with an infrared moisture meter is, for example, 4% (w/w) or less, preferably 2% (w/w) or less.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 4.0 - 6.0% (w/w) per 100% (w/w) of the formulation, and
(3) a lubricant in 2.0 - 10.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 5.0 - 6.0% (w/w) per 100% (w/w) of the formulation, and
(3) a lubricant in 4.0 - 6.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 4.5 - 6.0% (w/w) per 100% (w/w) of the formulation, and
(3) a lubricant in 2.0 - 10.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 4.0 - 6.0% (w/w) per 100% (w/w) of the formulation, and
(3) a lubricant in 4.0 - 10.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 4.5 - 6.0% (w/w) per 100% (w/w) of the formulation, and
(3) a lubricant in 4.0 - 10.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 5.0 - 5.5% (w/w) per 100% (w/w) of the formulation, and
(3) a lubricant in 4.0 - 10.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 5.0 - 6.0% (w/w) per 100% (w/w) of the formulation, and
(3) a lubricant in 4.0 - 10.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 5.0 - 5.5% (w/w) per 100% (w/w) of the formulation, and
(3) a lubricant in 2.0 - 10.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 4.5 - 6.0% (w/w) per 100% (w/w) of the formulation, and
(3) a lubricant in 2.0 - 6.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 4.5 - 6.0% (w/w) per 100% (w/w) of the formulation, and
(3) a lubricant in 4.0 - 6.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 5.0 - 5.5% (w/w) per 100% (w/w) of the formulation, and
(3) a lubricant in 2.0 - 6.0% (w/w) per 100% (w/w) of the formulation,
and optionally comprising (4) an excipient, (5) a disintegrant, and/or (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) hydroxypropyl cellulose in 5.0 - 6.0% (w/w) per 100% (w/w) of the formulation,
(3) sodium stearyl fumarate in 4.0 - 6.0% (w/w) per 100% (w/w) of the formulation,
(4) a combination of partially pregelatinized starch and D-mannitol, and
(5) croscarmellose sodium,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 15 - 38% (w/w) per 100% (w/w) of the formulation,
(2) hydroxypropyl cellulose in 5.0 - 6.0% (w/w) per 100% (w/w) of the formulation,
(3) sodium stearyl fumarate in 4.0 - 6.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 5.0 - 6.0% (w/w) per 100% (w/w) of the formulation,
(3) sodium stearyl fumarate in 4.0 - 6.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 15 - 38% (w/w) per 100% (w/w) of the formulation,
(2) hydroxypropyl cellulose in 4.5 - 6.0% (w/w) per 100% (w/w) of the formulation,
(3) sodium stearyl fumarate in 4.0 - 6.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 4.0 - 6.0% (w/w) per 100% (w/w) of the formulation,
(3) sodium stearyl fumarate in 2.0 - 10.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 4.0 - 6.0% (w/w) per 100% (w/w) of the formulation,
(3) sodium stearyl fumarate in 4.0 - 10.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 4.5 - 6.0% (w/w) per 100% (w/w) of the formulation,
(3) sodium stearyl fumarate in 2.0 - 10.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 4.5 - 6.0% (w/w) per 100% (w/w) of the formulation,
(3) sodium stearyl fumarate in 4.0 - 10.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 4.5 - 6.0% (w/w) per 100% (w/w) of the formulation,
(3) sodium stearyl fumarate in 2.0 - 6.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 4.5 - 6.0% (w/w) per 100% (w/w) of the formulation,
(3) sodium stearyl fumarate in 4.0 - 6.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38 % (w/w) per 100% (w/w) of the formulation,
(2) hydroxypropyl cellulose in 5.0 - 5.5% (w/w) per 100% (w/w) of the formulation,
(3) sodium stearyl fumarate in 2.0 - 10.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 5.0 - 5.5% (w/w) per 100% (w/w) of the formulation,
(3) sodium stearyl fumarate in 4.0 - 10.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 5.0 - 5.5% (w/w) per 100% (w/w) of the formulation,
(3) sodium stearyl fumarate in 2.0 - 6.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 38% (w/w) per 100% (w/w) of the formulation,
(2) hydroxypropyl cellulose in 5.0 - 5.5% (w/w) per 100% (w/w) of the formulation,
(3) sodium stearyl fumarate in 4.0 - 6.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 30% (w/w) per 100% (w/w) of the formulation,
(2) hydroxypropyl cellulose in 4.5 - 6.0% (w/w) per 100% (w/w) of the formulation,
(3) sodium stearyl fumarate in 2.0 - 10.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 30% (w/w) per 100% (w/w) of the formulation,
(2) hydroxypropyl cellulose in 4.5- 6.0% (w/w) per 100% (w/w) of the formulation,
(3) sodium stearyl fumarate in 4.0 - 10.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 30% (w/w) per 100% (w/w) of the formulation,
(2) hydroxypropyl cellulose in 4.5 - 6.0% (w/w) per 100% (w/w) of the formulation,
(3) sodium stearyl fumarate in 4.0 - 6.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### In an embodiment, the present disclosure provides an oral solid formulation comprising

(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof in 20 - 30% (w/w) per 100% (w/w) of the formulation,
(2) hydroxypropyl cellulose in 5.0 - 6.0% (w/w) per 100% (w/w) of the formulation,
(3) sodium stearyl fumarate in 4.0 - 6.0% (w/w) per 100% (w/w) of the formulation,
(4) a combination of partially pregelatinized starch and D-mannitol, and
(5) croscarmellose sodium in 0.5 - 20.0% (w/w) per 100% (w/w) of the formulation,
and optionally comprising (6) an additive, as needed.

### (2) Medical use of oral solid formulation

The medicament used herein has an antitumor effect by inhibiting the binding between "a MLL (mixed lineage leukemia) fusion protein that is fused with AF4, AF9, or the like, which is a representative fusion partner gene causing MLL leukemia", and menin, and thereby it is useful as an antitumor agent. Hence, the present invention relates to a pharmaceutical composition for treating cancer, therapeutic and/or preventive agent, preferably an oral solid formulation, comprising the present compound.

In the present invention, "prevention" refers to the act of administering the present medicament which is an active ingredient, to a healthy person who has not developed a disease or is in good health at the time of administration. A "preventive agent" is administered to such a healthy person, for example, with the purpose of preventing the onset of a disease. In particular, it is expected to be appropriate for people who have previously had symptoms of the disease or who are considered to be at increased risk of contracting a disease. "Treatment" refers to the act of administering the present medicament which is the active ingredient, to a person (patient) who has been diagnosed by a doctor as having a disease, and a "therapeutic agent" is something that is administered to such a patient, for example, to alleviate the disease or symptoms, prevent the disease or symptoms from worsening, or return the patient to the state before the onset of the disease. Furthermore, even if the purpose of administration is to prevent the disease or symptoms from worsening, it is still a therapeutic act if the recipient of the administration is a patient.

The "cancer" and "tumor" are used interchangeably, and the both mean malignant neoplasm, which encompasses carcinoma, sarcoma, and hematologic malignancy. The "cancer" and "tumor" include, for example, acute leukemia (including MLL acute leukemia, MLL partial tandem duplicate acute leukemia, NPM mutated acute leukemia, MOZ acute leukemia, NUP98 acute leukemia, and CALM acute leukemia), chronic lymphocytic leukemia, chronic myeloid leukemia, a myelodysplastic syndrome, polycythemia vera, malignant lymphoma (including B-cell lymphoma), myeloma (including multiple myeloma), brain tumor, head and neck cancer, esophageal cancer, thyroid cancer, small cell lung cancer, non-small cell lung cancer, breast cancer, gastric cancer, gallbladder and bile duct cancer, liver cancer, hepatocellular cancer, pancreatic cancer, colon cancer, rectal cancer, anal cancer, chorionepithelioma, endometrial cancer, cervical cancer, ovarian cancer, bladder cancer, urothelial cancer, renal cancer, renal cell cancer, prostate cancer, testicular tumor, testicular germ cell tumor , ovarian germ cell tumor, Wilms' tumor, malignant melanoma, neuroblastoma, osteosarcoma, Ewing's sarcoma, chondrosarcoma, soft tissue sarcoma, skin cancer, and the like. The above tumors may be accompanied by increased expression or mutation of specific genes. The tumors accompanied by increased expression of genes include, for example, tumors accompanied by high expression of HOXa gene cluster, tumors accompanied by high expression of MEIS gene cluster, and the like. The tumors accompanied by mutation of genes include tumors accompanied by p53 gain-of-function mutation and the like.

The present medicament can be used in combination with a "concomitant drug" to enhance its effect. The "concomitant drug" includes, for example, an alkylating antineoplastic agent, an antineoplastic antimetabolite, an antineoplastic antibiotic, a plant-derived antineoplastic agent, an antineoplastic platinum coordination complex, an antineoplastic camptothecin derivative, an antineoplastic tyrosine kinase inhibitor, an antineoplastic serine/threonine kinase inhibitor, an antineoplastic phospholipid kinase inhibitor, an antineoplastic monoclonal antibody, interferon, a biological response modifier, a hormonal agent, an immune checkpoint inhibitor, an epigenetic-targeting agent, a post-translational protein modification inhibitor, and other antineoplastic agents. The "combination drug" includes, for example, azacitidine, vorinostat, decitabine, romidepsin, idarubicin, daunorubicin, doxorubicin, enocitabine, cytarabine, mitoxantrone, thioguanine, etoposide, ifosfamide, cyclophosphamide, dacarbazine, temozolomide, nimustine, busulfan, procarbazine, melphalan, ranimustine, all-trans retinoic acid, tamibarotene, cisplatin, carboplatin, oxaliplatin, irinotecan, bleomycin, mitomycin C, methotrexate, paclitaxel, docetaxel, gemcitabine, tamoxifen, thiotepa, tegafur, fluorouracil, everolimus, temsirolimus, gefitinib, erlotinib, imatinib, crizotinib, osimertinib, afatinib, dasatinib, bosutinib, vandetanib, sunitinib, axitinib, pazopanib, lenvatinib, lapatinib, nilotinib, ibrutinib, ceritinib, alectinib, tofacitinib, baricitinib, ruxolitinib, olaparib, sorafenib, vemurafenib, dabrafenib, trametinib, palbociclib, bortezomib, carfilzomib, rituximab, cetuximab, trastuzumab, bevacizumab, panitumumab, nivolumab, atezolizumab, mogamulizumab, alemtuzumab, ofatumumab, ipilimumab, ramucirumab, brentuximab vedotin, gemtuzumab ozogamicin, and inotuzumab ozogamicin.

The timing of administration of the present medicament and a concomitant drug is not limited, and they may be administered to a subject simultaneously or at appropriate intervals. Furthermore, the present medicament may be administered as a combination drug with a concomitant drug, or the concomitant drug may be administered as a separate formulation from the present medicament, or may be administered via a different administration route. The dosage of a concomitant drug can be appropriately selected based on the dose used clinically. Furthermore, the mixing ratio of the present medicament and a concomitant drug can be appropriately selected depending on the subject of administration, the administration route, the target disease, symptoms, combination, etc.

The present compound or a pharmaceutically acceptable salt thereof, or a hydrate or solvate thereof may be administered orally or parenterally, with oral administration being preferred. The dosage varies depending on the administration method, the symptoms and age of the patient, etc., but is generally in the range of 0.01 to 30 mg/kg/day, preferably 0.05 to 20 mg/kg/day, and more preferably 0.1 to 10 mg/kg/day. Another preferred embodiment of the dosage is generally in the range of 0.01 mg to 1600 mg/day, preferably 0.1 mg to 1200 mg/day, more preferably 0.5 mg to 1000 mg/day, even more preferably 1 mg to 800 mg/day, and most preferably 5 mg to 600 mg/day. The daily dose may be administered once or several times daily, for example, 1, 2 or 3 doses each time.

### EXAMPLES

The present invention is explained in more detail in the following by referring to Examples of the present invention, however, the technical scope of the present invention should not be limited thereto. The following things may be changed without departing from the scope of the present disclosure. The compound names given in Examples and Tests below do not necessarily follow the IUPAC nomenclature.

In the present Examples, Tests and Comparative examples, unless otherwise specified, % in the solution indicates (wt %), and % in the particles indicates % (w/w).

The additives used in the Examples, Tests and Comparative examples were as follows, unless otherwise specified.

hydroxypropyl cellulose (HPC-L (6 - 10 mPa ·s¹⁾)) : NIPPON SODA CO., LTD.
polyvinyl alcohol (GOHSENOL^{™} EG-05P (4.5 - 6.1 mm²/s²⁾) )
hypromellose (TC-5^{™} R (6 mPa¹⁾)): Shin-Etsu Chemical Co., Ltd.
partially pregelatinized starch (PCS^{™} PC-10): Asahi Kasei Corporation
croscarmellose sodium (Ac-Di-Sol^{™}): DuPont
crospovidone (Kollidon^{™} CL): BASF
sodium starch glycolate (Primojel^{™}): DFE Pharma
mannitol (PEARLITOL 50C): Roquette
sodium stearyl fumarate (PRUV^{™}): JRS PHARMA GmbH & Co.KG
carnauba wax (Polishing wax 105): Nippon Wax Co., Ltd.
yellow ferric oxide S: Kishi Kasei Co., Ltd.
red ferric oxide S: Kishi Kasei Co., Ltd.

The OPADRY used in Examples is a film coating agent that does not affect dissolution.
1) Manufacturer catalog values for 2% solution at 20°C.
2) Manufacturer catalog values for 4% solution at 20°C.

### Example 1: Tablet comprising tartrate of the present compound (1)

### A. Composition of uncoated tablet comprising tartrate of the present compound

Granules, mixed powders and uncoated tablets having the compositions shown in Table 1-1 were prepared.

| Table 1-1. Composition (Example 1) | | | |
|---|---|---|---|
| | Ingredient | Amount | |
| | | (mg) | (wt%) |
| Granule ingredient | tartrate of the present compound | 50.04 | 27.8 |
| | D-mannitol | 74.16 | 41.2 |
| | partially pregelatinized starch | 36.0 | 20.0 |
| | croscarmellose sodium | 3.6 | 2.0 |
| | hydroxypropyl cellulose | 9.0 | 5.0 |
| | Subtotal of granule | 172.8 | 96.0 |
| Mixture ingredient | sodium stearyl fumarate | 7.2 | 4.0 |
| | Subtotal of mixed powder and uncoated tablet | 180.0 | 100.0 |

### B. Manufacturing process

### (1) Preparation of 5 wt% hydroxypropyl cellulose solution (1)

2850 g of purified water was placed in a SUS beaker, and 150 g of HPC-L was gradually added to the beaker while stirring to dissolve the mixture. After leaving the solution to stand overnight, it was stirred again to prepare a 5 wt% hydroxypropyl cellulose solution (1).

### (2) Preparation of granule comprising tartrate of the present compound

Granules comprising tartrate of the present compound were prepared using the ingredients and their amounts listed in Table 1-2 and under the preparation conditions listed in Table 1-3. First, tartrate of the present compound, D-mannitol, partially pregelatinized starch, and croscarmellose sodium in the amounts shown in Table 1-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (1) in the amount shown in Table 1-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 1-2. Preparation amount (Example 1, granules) | | |
|---|---|---|
| | Ingredient | Amount (g) |
| Granule ingredient | tartrate of the present compound | 198.7 |
| | D-mannitol | 294.4 |
| | partially pregelatinized starch | 142.9 |
| | croscarmellose sodium | 14.3 |
| | 5 wt% hydroxypropyl cellulose solution (1) | 714.7 |

| Table 1-3. Preparation condition (Example 1, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 72 - 73°C |
| Charge air volume | 0.6 - 0.7 m³/min |
| Spray speed | about 13 g/min |

### (3) Preparation of mixed powder comprising tartrate of the present compound

Mixed powder comprising tartrate of the present compound was prepared in the preparation amounts shown in Table 1-4. Granules comprising tartrate of the present compound in the amount shown in Table 1-4 were placed in a plastic bag, and then sodium stearyl fumarate in the amount shown in Table 1-4 was added thereto, and the contents of the bag were mixed (50 rotations vertically, 50 rotations horizontally, a total of 100 rotations).

| Table 1-4. Preparation amount (Example 1, mixed powder) | | |
|---|---|---|
| | Ingredient | Amount (g) |
| Mixed ingredients | granules comprising tartrate of the present compound | 309.97 |
| | Isodium stearyl fumarate | 12.92 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 1-5 so that each tablet comprises 50 mg of tartrate of the present compound.

| Table 1-5. Preparation condition (Example 1, uncoated tablet) | |
|---|---|
| Item | Preparation condition |
| Punch shape | Diameter: 8.0 mm, R: 12.0 mm |
| Turntable rotation speed | 20 rpm |
| Main pressure | 5.4 kN |

### (5) Preparation of film coating solution (1)

540.0 g of purified water was placed in an SUS tank, and 60.0 g of a coating component mixture comprising hypromellose, titanium oxide, propylene glycol, yellow ferric oxide, and red ferric oxide was gradually added thereto while stirring, and dispersed and dissolved. The solution was passed through a screen with an opening of 108 µm to give film coating solution (1).

### (6) Preparation of film-coated tablet comprising tartrate of the present compound

Film-coated tablets comprising tartrate of the present compound were prepared according to the conditions described in Table 1-6. First, 231.16 g of the uncoated tablets were placed in a coating machine (produced by FREUND CORPORATION, HC-LABO model), and heated under the conditions in Table 1-6 while the coating pan was rotated slightly until the exhaust temperature reached 45°C. Subsequently, while rotating the coating pan at 20 rpm, the film coating solution (1) was sprayed under the conditions in Table 1-6 to film-coat the tablets until each coating amount reached about 3.8 mg. Then, the film-coated tablets were dried until the exhaust temperature reached 50°C while the coating pan was rotated at 12 rpm. After the tablets were taken out, Polishing Wax 105 was added to the tablets and the mixture was mixed in a bag to be polished.

| Table 1-6. Preparation condition (Example 1, film-coated tablet) | |
|---|---|
| Item | Preparation condition |
| Spray gun bore | φ1.0 |
| Air cap hole diameter | φ2.5 |
| Spray air pressure | 0.15 MPa |
| Spray flow meter knob | full open |
| Adjustment screw for pattern air | 3 rotations |
| Static pressure inside pan | -50 Pa |
| Charge air temperature | 74 - 80°C |
| Charge air volume | 0.6 m³/min |
| Spray speed | 3 g/min |

### Example 2: Tablet comprising tartrate of the present compound (2)

### A. Composition of uncoated tablet comprising tartrate of the present compound

Granules, mixed powders and uncoated tablets having the compositions shown in Table 2-1 were prepared.

| Table 2-1. Composition (Example 2) | | | |
|---|---|---|---|
| | Ingredient | Amount | |
| | | (mg) | (wt%) |
| Granule ingredient | tartrate of the present compound | 50.0 | 20.0 |
| | D-mannitol | 126.25 | 50.5 |
| | partially pregelatinized starch | 50.0 | 20.0 |
| | croscarmellose sodium | 5.0 | 2.0 |
| | hydroxypropyl cellulose | 13.75 | 5.5 |
| | Subtotal of granule | 245.0 | 98.0 |
| Mixture ingredient | sodium stearyl fumarate | 5.0 | 2.0 |
| | Subtotal of mixed powder and uncoated tablet | 250.0 | 100.0 |

### B. Manufacturing process

### (1) Preparation of 5 wt% hydroxypropyl cellulose solution (2)

1900 g of purified water was placed in a SUS beaker, and 100 g of HPC-L was gradually added to the beaker while stirring to dissolve the mixture. After leaving the solution to stand overnight, it was stirred again to prepare a 5 wt% hydroxypropyl cellulose solution (2).

### (2) Preparation of granule comprising tartrate of the present compound

Granules comprising tartrate of the present compound were prepared using the ingredients and their amounts listed in Table 2-2 and under the preparation conditions listed in Table 2-3. First, tartrate of the present compound, D-mannitol, partially pregelatinized starch, and croscarmellose sodium in the amounts shown in Table 2-2 were charged into a tumbling fluid bed granulator dryer system (produced by POWREX CORPORATION, Multiplex MP-01 model) and mixed while fluidizing until the exhaust temperature reached 40°C. Subsequently, 5.5 wt% hydroxypropyl cellulose solution (2) in the amount shown in Table 2-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 2-2. Preparation amount (Example 2, granules) | | |
|---|---|---|
| | Ingredient | Amount (g) |
| Granule ingredient | tartrate of the present compound | 140 |
| | D-mannitol | 350 |
| | partially pregelatinized starch | 140 |
| | croscarmellose sodium | 14 |
| | 5 wt% hydroxypropyl cellulose solution (2) | 770.5 |

| Table 2-3. Preparation condition (Example 2, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 70 - 72°C |
| Charge air volume | 0.6 - 0.7 m³/min |
| Spray speed | about 13 g/min |

### (3) Preparation of mixed powder comprising tartrate of the present compound

Mixed powder comprising tartrate of the present compound was prepared in the preparation amounts shown in Table 2-4. Granules comprising tartrate of the present compound in the amount shown in Table 2-4 were placed in a plastic bag, and then sodium stearyl fumarate in the amount shown in Table 2-4 was added thereto, and the contents of the bag were mixed (50 rotations vertically, 50 rotations horizontally, a total of 100 rotations).

| Table 2-4. Preparation amount (Example 2, mixed powder) | | |
|---|---|---|
| | Ingredient | Amount (g) |
| Mixed ingredients | granules comprising tartrate of the present compound | 306.00 |
| | sodium stearyl fumarate | 6.24 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 2-5 so that each tablet comprises 50 mg of tartrate of the present compound.

| Table 2-5. Preparation condition (Example 2, uncoated tablet) | |
|---|---|
| Item | Preparation condition |
| Punch shape | Diameter: 9.0 mm, R: 13.0 mm |
| Turntable rotation speed | 20 rpm |
| Main pressure | 4.5 kN |

### Example 3: Tablet comprising tartrate of the present compound (3)

### A. Composition of uncoated tablet comprising tartrate of the present compound

Granules, mixed powders and uncoated tablets having the compositions shown in Table 3-1 were prepared.

| Table 3-1. Composition (Example 3) | | | |
|---|---|---|---|
| | Ingredient | Amount | |
| | | (mg) | (wt%) |
| Granule ingredient | tartrate of the present compound | 25.0 | 27.5 |
| | D-mannitol | 36.2 | 39.8 |
| | partially pregelatinized starch | 18.0 | 19.8 |
| | croscarmellose sodium | 1.8 | 2.0 |
| | hydroxypropyl cellulose | 4.5 | 4.9 |
| | Subtotal of granule | 85.5 | 94.0 |
| Mixture ingredient | sodium stearyl fumarate | 5.5 | 6.0 |
| | Subtotal of mixed powder and uncoated tablet | 91.0 | 100.0 |

### B. Manufacturing process

### (1) Preparation of 5 wt% hydroxypropyl cellulose solution (3)

2850 g of purified water was placed in a SUS beaker, and 150 g of HPC-L was gradually added to the beaker while stirring to dissolve the mixture. After leaving the solution to stand overnight, it was stirred again to prepare a 5 wt% hydroxypropyl cellulose solution (3).

### (2) Preparation of granule comprising tartrate of the present compound

Granules comprising tartrate of the present compound were prepared using the ingredients and their amounts listed in Table 3-2 and under the preparation conditions listed in Table 3-3. First, tartrate of the present compound, D-mannitol, partially pregelatinized starch, and croscarmellose sodium in the amounts shown in Table 3-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (3) in the amount shown in Table 3-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 3-2. Preparation amount (Example 3, granules) | | |
|---|---|---|
| | Ingredient | Amount (g) |
| Granule ingredient | tartrate of the present compound | 400 |
| | D-mannitol | 579.2 |
| | partially pregelatinized starch | 288 |
| | croscarmellose sodium | 28.8 |
| | 5 wt% hydroxypropyl cellulose solution (3) | 1440 |

| Table 3-3. Preparation condition (Example 3, granules) | |
|---|---|
| Item | Preparation condition |
| Container used | standard container |
| Gun bore | 1.0 mm |
| Gun height | up |
| Spray type | top-spray |
| Spray pressure | 0.12 MPa |
| Charge air temperature | 80°C |
| Charge air volume | 0.7 - 0.8 m³/min |
| Spray speed | about 15 g/min |

### (3) Preparation of mixed powder comprising tartrate of the present compound

Mixed powder comprising tartrate of the present compound was prepared in the preparation amounts shown in Table 3-4. Granules comprising tartrate of the present compound in the amount shown in Table 3-4 were placed in a 10 L V-shaped mixer (Tsutsui Scientific Instruments Co., Ltd., S-5 model), and then sodium stearyl fumarate in the amount shown in Table 3-4 was added thereto, and the contents were mixed in the mixer for 10 minutes at 40 rpm.

| Table 3-4. Preparation amount (Example 3, mixed powder) | | |
|---|---|---|
| | Ingredient | Amount (g) |
| Mixed ingredients | granules comprising tartrate of the present compound | 650.00 |
| | sodium stearyl fumarate | 41.49 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 3-5 so that each tablet comprises 25 mg of tartrate of the present compound.

| Table 3-5. Preparation condition (Example 3, uncoated tablet) | |
|---|---|
| Item | Preparation condition |
| Punch shape | Diameter: 6.0 mm, R: 8.0 mm |
| Turntable rotation speed | 20 rpm |
| Main pressure | 3.9 kN |

### (5) Preparation of film coating solution (2)

1800.00 g of purified water was weighed out. To a portion of the purified water were added 0.32 g of yellow ferric oxide and 0.16 g of red ferric oxide, and the mixture was dispersed using Robomics (T.K. HOMO MIXER MARK 2.5 type). 199.52 g of a coating component mixture comprising hypromellose, titanium oxide, and propylene glycol was gradually added thereto while stirring, and dispersed and dissolved. To the solution was added a solution dispersed with a pigment in a portion of the purified water. Finally, the solution was passed through a screen with an opening of 108 µm to give film coating solution (2).

### (6) Preparation of film-coated tablet comprising tartrate of the present compound

Film-coated tablets comprising tartrate of the present compound were prepared according to the conditions described in Table 3-6. First, 400.00 g of the uncoated tablets were placed in a coating machine (produced by FREUND CORPORATION, HC-LABO model), and heated under the conditions in Table 3-6 while the coating pan was rotated slightly until the exhaust temperature reached 45°C. Subsequently, while rotating the coating pan at 20 rpm, the film coating solution (2) was sprayed under the conditions in Table 3-6 to film-coat the tablets until each coating amount reached about 2.4 mg. Then, the film-coated tablets were dried until the exhaust temperature reached 50°C while the coating pan was rotated at 12 rpm. After the tablets were taken out, Polishing Wax 105 was added to the tablets and the mixture was mixed in a bag to be polished.

| Table 3-6. Preparation condition (Example 3, film-coated tablet) | |
|---|---|
| Item | Preparation condition |
| Spray gun bore | φ1.0 |
| Air cap hole diameter | φ2.5 |
| Spray air pressure | 0.15 MPa |
| Spray flow meter knob | full open |
| Adjustment screw for pattern air | 3 rotations |
| Static pressure inside pan | -50 Pa |
| Charge air temperature | 70°C |
| Charge air volume | 0.6 m³/min |
| Spray speed | 2 g/min |

### Example 4: Tablet comprising tartrate of the present compound (4)

### A. Composition of uncoated tablet comprising tartrate of the present compound

Granules, mixed powders and uncoated tablets having the compositions shown in Table 4-1 were prepared.

| Table 4-1. Composition (Example 4) | | | |
|---|---|---|---|
| | Ingredient | Amount | |
| | | (mg) | (wt%) |
| Granule ingredient | tartrate of the present compound | 50.0 | 27.8 |
| | D-mannitol | 54.4 | 30.2 |
| | partially pregelatinized starch | 54.0 | 30.0 |
| | croscarmellose sodium | 5.4 | 3.0 |
| | hydroxypropyl cellulose | 9.0 | 5.0 |
| | Subtotal of granule | 172.8 | 96.0 |
| Mixture ingredient | sodium stearyl fumarate | 7.2 | 4.0 |
| | Subtotal of mixed powder and uncoated tablet | 180.0 | 100 |

### B. Manufacturing process

### (1) Preparation of 5 wt% hydroxypropyl cellulose solution (4)

2850 g of purified water was placed in a SUS beaker, and 150 g of HPC-L was gradually added to the beaker while stirring to dissolve the mixture. After leaving the solution to stand overnight, it was stirred again to prepare a 5 wt% hydroxypropyl cellulose solution (4).

### (2) Preparation of granule comprising tartrate of the present compound

Granules comprising tartrate of the present compound were prepared using the ingredients and their amounts listed in Table 4-2 and under the preparation conditions listed in Table 4-3. First, tartrate of the present compound, D-mannitol, partially pregelatinized starch, and croscarmellose sodium in the amounts shown in Table 4-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (4) in the amount shown in Table 4-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 4-2. Preparation amount (Example 4, granules) | | |
|---|---|---|
| | Ingredient | Amount (g) |
| Granule ingredient | tartrate of the present compound | 200 |
| | D-mannitol | 217.6 |
| | partially pregelatinized starch | 216 |
| | croscarmellose sodium | 21.6 |
| | 5 wt% hydroxypropyl cellulose solution (4) | 720.0 |

| Table 4-3. Preparation condition (Example 4, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 73°C |
| Charge air volume | 0.7 - 0.8 m³/min |
| Spray speed | about 13 g/min |

### (3) Preparation of mixed powder comprising tartrate of the present compound

Mixed powder comprising tartrate of the present compound was prepared in the preparation amounts shown in Table 4-4. Granules comprising tartrate of the present compound in the amount shown in Table 4-4 were placed in a 10 L V-shaped mixer (Tsutsui Scientific Instruments Co., Ltd., S-5 model), and then sodium stearyl fumarate in the amount shown in Table 4-4 was added thereto, and the contents were mixed in the mixer for 10 minutes at 40 rpm.

| Table 4-4. Preparation amount (Example 4, mixed powder) | | |
|---|---|---|
| | Ingredient | Amount (g) |
| Mixed ingredients | granules comprising tartrate of the present compound | 630.00 |
| | sodium stearyl fumarate | 26.25 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 4-5 so that each tablet comprises 50 mg of tartrate of the present compound.

| Table 4-5. Preparation condition (Example 4, uncoated tablet) | |
|---|---|
| Item | Preparation condition |
| Punch shape | Diameter: 8.0 mm, R: 12.0 mm |
| Turntable rotation speed | 20 rpm |
| Main pressure | 7.0 kN |

### (5) Preparation of film coating solution (3)

900.00 g of purified water was weighed out. To a portion of the purified water was added 0.24 g of yellow ferric oxide, and the mixture was dispersed using Robomics (T.K. HOMO MIXER MARK 2.5 type). 99.76 g of a coating component mixture comprising hypromellose, titanium oxide, and propylene glycol was gradually added thereto while stirring, and dispersed and dissolved. To the solution was added a solution dispersed with a pigment in a portion of the purified water. Finally, the solution was passed through a screen with an opening of 108 µm to give film coating solution (3).

### (6) Preparation of film-coated tablet comprising tartrate of the present compound

Film-coated tablets comprising tartrate of the present compound were prepared according to the conditions described in Table 4-6. First, 367.43 g of the uncoated tablets were placed in a coating machine (produced by FREUND CORPORATION, HC-LABO model), and heated under the conditions in Table 4-6 while the coating pan was rotated slightly until the exhaust temperature reached 45°C. Subsequently, while rotating the coating pan at 20 rpm, the film coating solution (3) was sprayed under the conditions in Table 4-6 to film-coat the tablets until each coating amount reached about 3.8 mg. Then, the film-coated tablets were dried until the exhaust temperature reached 50°C while the coating pan was rotated at 12 rpm. After the tablets were taken out, Polishing Wax 105 was added to the tablets and the mixture was mixed in a bag to be polished.

| Table 4-6. Preparation condition (Example 4, film-coated tablet) | |
|---|---|
| Item | Preparation condition |
| Spray gun bore | φ1.0 |
| Air cap hole diameter | φ2.5 |
| Spray air pressure | 0.15 MPa |
| Spray flow meter knob | full open |
| Adjustment screw for pattern air | 3 rotations |
| Static pressure inside pan | -50 Pa |
| Charge air temperature | 70°C |
| Charge air volume | 0.6 m³/min |
| Spray speed | 2 g/min |

### Example 5: Tablet comprising tartrate of the present compound (5)

### A. Composition of uncoated tablet comprising tartrate of the present compound

Granules, mixed powders and uncoated tablets having the compositions shown in Table 5-1 were prepared.

| Table 5-1. Composition (Example 5) | | | |
|---|---|---|---|
| | Ingredient | Amount | |
| | | (mg) | (wt%) |
| Granule ingredient | tartrate of the present compound | 125.0 | 35.7 |
| | D-mannitol | 116.5 | 33.3 |
| | partially pregelatinized starch | 70.0 | 20.0 |
| | croscarmellose sodium | 7.0 | 2.0 |
| | hydroxypropyl cellulose | 17.5 | 5.0 |
| | Subtotal of granule | 336.0 | 96.0 |
| Mixture | sodium stearyl fumarate | 14.0 | 4.0 |
| ingredient | Subtotal of mixed powder and uncoated tablet | 350.0 | 100.0 |

### B. Manufacturing process

### (1) Preparation of 5 wt% hydroxypropyl cellulose solution (5)

2850 g of purified water was placed in a SUS beaker, and 150 g of HPC-L was gradually added to the beaker while stirring to dissolve the mixture. After leaving the solution to stand overnight, it was stirred again to prepare a 5 wt% hydroxypropyl cellulose solution (5).

### (2) Preparation of granule comprising tartrate of the present compound

Granules comprising tartrate of the present compound were prepared using the ingredients and their amounts listed in Table 5-2 and under the preparation conditions listed in Table 5-3. First, tartrate of the present compound, D-mannitol, partially pregelatinized starch, and croscarmellose sodium in the amounts shown in Table 5-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (1) in the amount shown in Table 5-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 5-2. Preparation amount (Example 5, granules) | | |
|---|---|---|
| | Ingredient | Amount (g) |
| Granule ingredient | tartrate of the present compound | 500 |
| | D-mannitol | 466 |
| | partially pregelatinized starch | 280 |
| | croscarmellose sodium | 28 |
| | 5 wt% hydroxypropyl cellulose solution (5) | 1400.0 |

| Table 5-3. Preparation condition (Example 5, granules) | |
|---|---|
| Item | Preparation condition |
| Container used | standard container |
| Gun bore | 1.0 mm |
| Gun height | up |
| Spray type | top-spray |
| Spray pressure | 0.12 MPa |
| Charge air temperature | 80°C |
| Charge air volume | 0.7 - 0.8 m³/min |
| Spray speed | about 15 g/min |

### (3) Preparation of mixed powder comprising tartrate of the present compound

Mixed powder comprising tartrate of the present compound was prepared in the preparation amounts shown in Table 5-4. Granules comprising tartrate of the present compound in the amount shown in Table 5-4 were placed in a 10 L V-shaped mixer (Tsutsui Scientific Instruments Co., Ltd., S-5 model), and then sodium stearyl fumarate in the amount shown in Table 5-4 was added thereto, and the contents were mixed in the mixer for 10 minutes at 40 rpm.

| ITable 5-4. Preparation amount (Example 5, mixed powder) | | |
|---|---|---|
| | Ingredient | Amount (g) |
| Mixed ingredients | granules comprising tartrate of the present compound | 1277.97 |
| | sodium stearyl fumarate | 53.25 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 5-5 so that each tablet comprises 125 mg of tartrate of the present compound.

| Table 5-5. Preparation condition (Example 5, uncoated tablet) | |
|---|---|
| Item | Preparation condition |
| Punch shape | Diameter: 14.0 x 6.5 mm, R: 5.04 mm |
| Turntable rotation speed | 15 rpm |
| Main pressure | 8.0 kN |

### (5) Preparation of film coating solution (4)

900.00 g of purified water was weighed out. To a portion of the purified water was added 0.24 g of red ferric oxide, and the mixture was dispersed using Robomics (T.K. HOMO MIXER MARK 2.5 type). 99.76 g of a coating component mixture comprising hypromellose, titanium oxide, and propylene glycol was gradually added thereto while stirring, and dispersed and dissolved. To the solution was added a solution dispersed with a pigment in a portion of the purified water. Finally, the solution was passed through a screen with an opening of 108 µm to give film coating solution (4).

### (6) Preparation of film-coated tablet comprising tartrate of the present compound

Film-coated tablets comprising tartrate of the present compound were prepared according to the conditions described in Table 5-6. First, 366.80 g of the uncoated tablets were placed in a coating machine (produced by FREUND CORPORATION, HC-LABO model), and heated under the conditions in Table 5-6 while the coating pan was rotated slightly until the exhaust temperature reached 45°C. Subsequently, while rotating the coating pan at 20 rpm, the film coating solution (4) was sprayed under the conditions in Table 5-6 to film-coat the tablets until each coating amount reached about 6.0 mg. Then, the film-coated tablets were dried until the exhaust temperature reached 50°C while the coating pan was rotated at 12 rpm. After the tablets were taken out, Polishing Wax 105 was added to the tablets and the mixture was mixed in a bag to be polished.

| Table 5-6. Preparation condition (Example 5, film-coated tablet) | |
|---|---|
| Item | Preparation condition |
| Spray gun bore | φ1.0 |
| Air cap hole diameter | φ2.5 |
| Spray air pressure | 0.15MPa |
| Spray flow meter knob | full open |
| Adjustment screw for pattern air | 3 rotations |
| Static pressure inside pan | -50 Pa |
| Charge air temperature | 70°C |
| Charge air volume | 0.6 m³/min |
| Spray speed | 2 g/min |

### Example 6: Tablet comprising tartrate of the present compound (6)

### A. Composition of uncoated tablet comprising tartrate of the present compound

Granules, mixed powders and uncoated tablets having the compositions shown in Table 6-1 were prepared.

| Table 6-1. Composition (Example 6) | | | |
|---|---|---|---|
| | Ingredient | Amount | |
| | | (mg) | (wt%) |
| Granule ingredient | tartrate of the present compound | 50.0 | 27.8 |
| | D-mannitol | 72.4 | 40.2 |
| | partially pregelatinized starch | 36.0 | 20.0 |
| | croscarmellose sodium | 3.6 | 2.0 |
| | hydroxypropyl cellulose | 10.8 | 6.0 |
| | Subtotal of granule | 172.8 | 96.0 |
| Mixture ingredient | sodium stearyl fumarate | 7.2 | 4.0 |
| | Subtotal of mixed powder and uncoated tablet | 180.0 | 100.0 |

### B. Manufacturing process

### (1) Preparation of 5 wt% hydroxypropyl cellulose solution (6)

2850 g of purified water was placed in a SUS beaker, and 150 g of HPC-L was gradually added to the beaker while

stirring to dissolve the mixture. After leaving the solution to stand overnight, it was stirred again to prepare a 5 wt% hydroxypropyl cellulose solution (6).

### (2) Preparation of granule comprising tartrate of the present compound

Granules comprising tartrate of the present compound were prepared using the ingredients and their amounts listed in Table 6-2 and under the preparation conditions listed in Table 6-3. First, tartrate of the present compound, D-mannitol, partially pregelatinized starch, and croscarmellose sodium in the amounts shown in Table 6-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (6) in the amount shown in Table 6-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 6-2. Preparation amount (Example 6, granules) | | |
|---|---|---|
| | Ingredient | Amount (g) |
| Granule ingredient | tartrate of the present compound | 200 |
| | D-mannitol | 289.6 |
| | partially pregelatinized starch | 144 |
| | croscarmellose sodium | 14.4 |
| | 5 wt% hydroxypropyl cellulose solution (6) | 864.0 |

| Table 6-3. Preparation condition (Example 6, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 73°C |
| Charge air volume | 0.7 - 0.8 m³/min |
| Spray speed | about 13 g/min |

### (3) Preparation of mixed powder comprising tartrate of the present compound

Mixed powder comprising tartrate of the present compound was prepared in the preparation amounts shown in Table 6-4. Granules comprising tartrate of the present compound in the amount shown in Table 6-4 were placed in a 10 L V-shaped mixer (Tsutsui Scientific Instruments Co., Ltd., S-5 model), and then sodium stearyl fumarate in the amount shown in Table 6-4 was added thereto, and the contents were mixed in the mixer for 10 minutes at 40 rpm.

| Table 6-4. Preparation amount (Example 6, mixed powder) | | |
|---|---|---|
| | Ingredient | Amount (g) |
| Mixed ingredients | granules comprising tartrate of the present compound | 636.72 |
| | sodium stearyl fumarate | 26.53 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 6-5 so that each tablet comprises 50 mg of tartrate of the present compound.

| Table 6-5. Preparation condition (Example 6, uncoated tablet) | |
|---|---|
| Item | Preparation condition |
| Punch shape | Diameter: 8.0 mm, R: 12.0 mm |
| Turntable rotation speed | 20 rpm |
| Main pressure | 5.0 kN |

### Example 7: Tablet comprising tartrate of the present compound (7)

### A. Composition of uncoated tablet comprising tartrate of the present compound

Granules, mixed powders and uncoated tablets having the compositions shown in Table 7-1 were prepared.

| Table 7-1. Composition (Example 7) | | | |
|---|---|---|---|
| | Ingredient | Amount | |
| | | (mg) | (wt%) |
| Granule ingredient | tartrate of the present compound | 50.0 | 27.8 |
| | D-mannitol | 76.0 | 42.2 |
| | partially pregelatinized starch | 36.0 | 20.0 |
| | croscarmellose sodium | 3.6 | 2.0 |
| | hydroxypropyl cellulose | 7.2 | 4.0 |
| | Subtotal of granule | 172.8 | 96.0 |
| Mixture ingredient | sodium stearyl fumarate | 7.2 | 4.0 |
| | Subtotal of mixed powder and uncoated tablet | 180 | 100 |

### B. Manufacturing process

### (1) Preparation of 5 wt% hydroxypropyl cellulose solution (7)

2850 g of purified water was placed in a SUS beaker, and 150 g of HPC-L was gradually added to the beaker while stirring to dissolve the mixture. After leaving the solution to stand overnight, it was stirred again to prepare a 5 wt% hydroxypropyl cellulose solution (7).

### (2) Preparation of granule comprising tartrate of the present compound

Granules comprising tartrate of the present compound were prepared using the ingredients and their amounts listed in Table 7-2 and under the preparation conditions listed in Table 7-3. First, tartrate of the present compound, D-mannitol, partially pregelatinized starch, and croscarmellose sodium in the amounts shown in Table 7-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (7) in the amount shown in Table 7-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 7-2. Preparation amount (Example 7, granules) | | |
|---|---|---|
| | Ingredient | Amount (g) |
| Granule ingredient | tartrate of the present compound | 200 |
| | D-mannitol | 304 |
| | partially pregelatinized starch | 144 |
| | croscarmellose sodium | 14.4 |
| | 5 wt% hydroxypropyl cellulose solution (7) | 576.0 |

| Table 7-3. Preparation condition (Example 7, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 73°C |
| Charge air volume | 0.6 - 0.8 m³/min |
| Spray speed | about 13 g/min |

### (3) Preparation of mixed powder comprising tartrate of the present compound

Mixed powder comprising tartrate of the present compound was prepared in the preparation amounts shown in Table 7-4. Granules comprising tartrate of the present compound in the amount shown in Table 7-4 were placed in a 10 L V-shaped mixer (Tsutsui Scientific Instruments Co., Ltd., S-5 model), and then sodium stearyl fumarate in the amount shown in Table 7-4 was added thereto, and the contents were mixed in the mixer for 10 minutes at 40 rpm.

| Table 7-4. Preparation amount (Example 7, mixed powder) | | |
|---|---|---|
| | Ingredient | Amount (g) |
| Mixed ingredients | granules comprising tartrate of the present compound | 632.51 |
| | sodium stearyl fumarate | 26.35 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 7-5 so that each tablet comprises 50 mg of tartrate of the present compound.

| Table 7-5. Preparation condition (Example 7, uncoated tablet) | |
|---|---|
| Item | Preparation condition |
| Punch shape | Diameter: 8.0 mm, R: 12.0 mm |
| Turntable rotation speed | 20 rpm |
| Main pressure | 5.0 kN |

### Example 8: Tablet comprising tartrate of the present compound (8)

### A. Composition of uncoated tablet comprising tartrate of the present compound

Granules, mixed powders and uncoated tablets having the compositions shown in Table 8-1 were prepared.

| Table 8-1. Composition (Example 8) | | | |
|---|---|---|---|
| | Ingredient | Amount | |
| | | (mg) | (wt%) |
| Granule ingredient | tartrate of the present compound | 25.0 | 27.8 |
| | D-mannitol | 36.2 | 40.2 |
| | partially pregelatinized starch | 18.0 | 20.0 |
| | croscarmellose sodium | 1.8 | 2.0 |
| | hydroxypropyl cellulose | 4.5 | 5.0 |
| | Subtotal of granule | 85.5 | 95.0 |
| Mixture ingredient | sodium stearyl fumarate | 4.5 | 5.0 |
| | Subtotal of mixed powder and uncoated tablet | 90.0 | 100.0 |

### B. Manufacturing process

### (1) Preparation of 5 wt% hydroxypropyl cellulose solution (8)

2850 g of purified water was placed in a SUS beaker, and 150 g of HPC-L was gradually added to the beaker while stirring to dissolve the mixture. After leaving the solution to stand overnight, it was stirred again to prepare a 5 wt% hydroxypropyl cellulose solution (8).

### (2) Preparation of granule comprising tartrate of the present compound

Granules comprising tartrate of the present compound were prepared using the ingredients and their amounts listed in Table 8-2 and under the preparation conditions listed in Table 8-3. First, tartrate of the present compound, D-mannitol, partially pregelatinized starch, and croscarmellose sodium in the amounts shown in Table 8-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (8) in the amount shown in Table 8-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 8-2. Preparation amount (Example 8, granules) | | |
|---|---|---|
| | Ingredient | Amount (g) |
| Granule ingredient | tartrate of the present compound | 400 |
| | D-mannitol | 579.2 |
| | partially pregelatinized starch | 288 |
| | croscarmellose sodium | 28.8 |
| | 5 wt% hydroxypropyl cellulose solution (8) | 1440 |

| Table 8-3. Preparation condition (Example 8, granules) | |
|---|---|
| Item | Preparation condition |
| Container used | standard container |
| Gun bore | 1.0 mm |
| Gun height | up |
| Spray type | top-spray |
| Spray pressure | 0.12 MPa |
| Charge air temperature | 80°C |
| Charge air volume | 0.7 - 0.8 m³/min |
| Spray speed | about 15 g/min |

### (3) Preparation of mixed powder comprising tartrate of the present compound

Mixed powder comprising tartrate of the present compound was prepared in the preparation amounts shown in Table 8-4. Granules comprising tartrate of the present compound in the amount shown in Table 8-4 were placed in a 10 L V-shaped mixer (Tsutsui Scientific Instruments Co., Ltd., S-5 model), and then sodium stearyl fumarate in the amount shown in Table 8-4 was added thereto, and the contents were mixed in the mixer for 10 minutes at 40 rpm.

| Table 8-4. Preparation amount (Example 8, mixed powder) | | |
|---|---|---|
| I | Ingredient | Amount (g) |
| Mixed ingredients | granules comprising tartrate of the present compound | 650.00 |
| | sodium stearyl fumarate | 34.21 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 8-5 so that each tablet comprises 25 mg of tartrate of the present compound.

| Table 8-5. Preparation condition (Example 8, uncoated tablet) | |
|---|---|
| Item | Preparation condition |
| Punch shape | Diameter: 6.0 mm, R: 8.0 mm |
| Turntable rotation speed | 20 rpm |
| Main pressure | 3.7 kN |

### (5) Preparation of film coating solution (5)

1800.00 g of purified water was weighed out. To a portion of the purified water were added 0.32 g of yellow ferric oxide and 0.16 g of red ferric oxide, and the mixture was dispersed using Robomics (T.K. HOMO MIXER MARK 2.5 type). 199.52 g of a coating component mixture comprising hypromellose, titanium oxide, and propylene glycol was gradually added thereto while stirring, and dispersed and dissolved. To the solution was added a solution dispersed with a pigment in a portion of the purified water. Finally, the solution was passed through a screen with an opening of 108 µm to give film coating solution (5).

### (6) Preparation of film-coated tablet comprising tartrate of the present compound

Film-coated tablets comprising tartrate of the present compound were prepared according to the conditions described in Table 8-6. First, 400.06 g of the uncoated tablets were placed in a coating machine (produced by FREUND CORPORATION, HC-LABO model), and heated under the conditions in Table 8-6 while the coating pan was rotated slightly until the exhaust temperature reached 45°C. Subsequently, while rotating the coating pan at 20 rpm, the film coating solution (5) was sprayed under the conditions in Table 8-6 to film-coat the tablets until each coating amount reached about 2.4 mg. Then, the film-coated tablets were dried until the exhaust temperature reached 50°C while the coating pan was rotated at 12 rpm. After the tablets were taken out, Polishing Wax 105 was added to the tablets and the mixture was mixed in a bag to be polished.

| Table 8-6. Preparation condition (Example 8, film-coated tablet) | |
|---|---|
| Item | Preparation condition |
| Spray gun bore | φ1.0 |
| Air cap hole diameter | φ2.5 |
| Spray air pressure | 0.15 MPa |
| Spray flow meter knob | full open |
| Adjustment screw for pattern air | 3 rotations |
| Static pressure inside pan | -50 Pa |
| Charge air temperature | 70°C |
| Charge air volume | 0.6 m³/min |
| Spray speed | 2 g/min |

### Example 9: Tablet comprising tartrate of the present compound (9)

### A. Composition of uncoated tablet comprising tartrate of the present compound

Granules, mixed powders and uncoated tablets having the compositions shown in Table 9-1 were prepared.

| Table 9-1. Composition (Example 9) | | | |
|---|---|---|---|
| | Ingredient | Amount | |
| | | (mg) | (wt%) |
| Granule ingredient | tartrate of the present compound | 124.7 | 37.8 |
| | D-mannitol | 86.5 | 26.2 |
| | partially pregelatinized starch | 66.0 | 20.0 |
| | croscarmellose sodium | 13.2 | 4.0 |
| | hydroxypropyl cellulose | 19.8 | 6.0 |
| | Subtotal of granule | 310.2 | 94.0 |
| Mixture ingredient | sodium stearyl fumarate | 19.8 | 6.0 |
| | Subtotal of mixed powder and uncoated tablet | 330.0 | 100.0 |

### B. Manufacturing process

### (1) Preparation of 5 wt% hydroxypropyl cellulose solution (9)

3325 g of purified water was placed in a SUS beaker, and 175 g of HPC-L was gradually added to the beaker while stirring to dissolve the mixture. After leaving the solution to stand overnight, it was stirred again to prepare a 5 wt% hydroxypropyl cellulose solution (9).

### (2) Preparation of granule comprising tartrate of the present compound

Granules comprising tartrate of the present compound were prepared using the ingredients and their amounts listed in Table 9-2 and under the preparation conditions listed in Table 9-3. First, tartrate of the present compound, D-mannitol, partially pregelatinized starch, and croscarmellose sodium in the amounts shown in Table 9-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (9) in the amount shown in Table 9-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Tablet 9-2. Preparation amount (Example 9, granules) | | |
|---|---|---|
| | Ingredient | Amount (g) |
| Granule ingredient | tartrate of the present compound | 280.0 |
| | D-mannitol | 194.1 |
| | partially pregelatinized starch | 148.1 |
| | croscarmellose sodium | 29.6 |
| | 5 wt% hydroxypropyl cellulose solution (9) | 888.8 |

| Table 9-3. Preparation condition (Example 9, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 73°C |
| Charge air volume | 0.6 - 0.7 m³/min |
| Spray speed | about 13 g/min |

### (3) Preparation of mixed powder comprising tartrate of the present compound

Mixed powder comprising tartrate of the present compound was prepared in the preparation amounts shown in Table 9-4. Granules comprising tartrate of the present compound in the amount shown in Table 9-4 were placed in a plastic bag, and then sodium stearyl fumarate in the amount shown in Table 9-4 was added thereto, and the contents of the bag were mixed (50 rotations vertically, 50 rotations horizontally, a total of 100 rotations).

| Table 9-4. Preparation amount (Example 9, mixed powder) | | |
|---|---|---|
| | Ingredient | Amount (g) |
| Mixed ingredients | granules comprising tartrate of the present compound | 633.30 |
| | sodium stearyl fumarate | 40.42 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 9-5 so that each tablet comprises 125 mg of tartrate of the present compound.

| Table 9-5. Preparation condition (Example 9, uncoated tablet) | |
|---|---|
| Item | Preparation condition |
| Punch shape | Diameter: 14.0 x 6.5 mm, R: 5.04 mm |
| Turntable rotation speed | 15 rpm |
| Main pressure | 8.2 kN |

### Example 10: Tablet comprising tartrate of the present compound (10)

### A. Composition of uncoated tablet comprising tartrate of the present compound

Granules, mixed powders and uncoated tablets having the compositions shown in Table 10-1 were prepared.

| Table 10-1. Composition (Example 10) | | | |
|---|---|---|---|
| | Ingredient | Amount | |
| | | (mg) | (wt%) |
| Granule ingredient | tartrate of the present compound | 125.0 | 27.8 |
| | D-mannitol | 185.5 | 41.2 |
| | partially pregelatinized starch | 90.0 | 20.0 |
| | croscarmellose sodium | 9.0 | 2.0 |
| | hydroxypropyl cellulose | 22.5 | 5.0 |
| | Subtotal of granule | 432.0 | 96.0 |
| Mixture ingredient | sodium stearyl fumarate | 18.0 | 4.0 |
| | Subtotal of mixed powder and uncoated tablet | 450.0 | 100.0 |

### B. Manufacturing process

### (1) Preparation of 5 wt% hydroxypropyl cellulose solution (10)

24624 g of purified water was placed in a SUS beaker, and 1296 g of HPC-L was gradually added to the beaker while stirring to dissolve the mixture. After leaving the solution to stand overnight, it was stirred again to prepare a 5 wt% hydroxypropyl cellulose solution (10).

### (2) Preparation of granule comprising tartrate of the present compound

Granules comprising tartrate of the present compound were prepared using the ingredients and their amounts listed in Table 10-2 and under the preparation conditions listed in Table 10-3. First, tartrate of the present compound, D-mannitol, partially pregelatinized starch, and croscarmellose sodium in the amounts shown in Table 10-2 were charged into a tumbling fluid bed granulator dryer system (produced by FREUND CORPORATION, NFLF-30SJC model) and mixed while fluidizing until the exhaust temperature reached 40°C. Subsequently, 5 wt% hydroxypropyl cellulose solution (10) in the amount shown in Table 10-2 was sprayed onto the mixture while fluidizing. Then, the mixture was dried while being fluidized until the exhaust temperature reached 45°C, and then removed from the container. The removed granules were passed through Conical Mill for dry granulation to obtain granules comprising tartrate salt of the present compound.

| Table 10-2. Preparation amount (Example 10, granules) | | |
|---|---|---|
| | Ingredient | Amount (g) |
| Granule ingredient | tartrate of the present compound | 6000 |
| | D-mannitol | 8904 |
| | partially pregelatinized starch | 4320 |
| | croscarmellose sodium | 432 |
| | 5 wt% hydroxypropyl cellulose solution (10) | 21600 |

| Table 10-3. Preparation condition (Example 10, granules) | |
|---|---|
| Item | Preparation condition |
| Container used | standard container |
| Gun bore | 2.0 mm |
| Spray pressure | 0.4 - 0.5 MPa |
| Charge air temperature | 80°C |
| Charge air volume | 8.0 - 10.0 m³/min |
| Spray speed | about 200 g/min |

### (3) Preparation of mixed powder comprising tartrate of the present compound

Mixed powder comprising tartrate of the present compound was prepared in the preparation amounts shown in Table 10-4. Granules comprising tartrate of the present compound in the amount shown in Table 10-4 were placed in a 110 L hopper bottle, and then sodium stearyl fumarate in the amount shown in Table 10-4 was added thereto, and the contents in the bottle were mixed for 15 minutes at 20 rpm using a mixer (produced by Yamazaki Metal Industry, MB02-001 type) .

| Table 10-4. Preparation amount (Example 10, mixed powder) | | |
|---|---|---|
| | Ingredient | Amount (g) |
| Mixed ingredients | granules comprising tartrate of the present compound | 19900 |
| | sodium stearyl fumarate | 829 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 10-5 so that each tablet comprises 125 mg of tartrate of the present compound.

| Table 10-5. Preparation condition (Example 10, uncoated tablet) | |
|---|---|
| Item | Preparation condition |
| Punch shape | Diameter: 16.0 x 6.5 mm, R:4.1 mm |
| Turntable rotation speed | 20 rpm |
| Main pressure | 15.0 kN |

### (5) Preparation of film coating solution (6)

10800 g of purified water was weighed out. To a portion of the purified water were added 9.6 g of yellow ferric oxide and 4.8 g of red ferric oxide, and the mixture was dispersed using Robomics (T.K. HOMOJETTER O type, HOMO MIXER MARK II 40 type). 1185.6 g of a coating component mixture comprising hypromellose, titanium oxide, and propylene glycol was gradually added thereto while stirring, and dispersed and dissolved. To the solution was added a solution dispersed with a pigment in a portion of the purified water. Finally, the solution was passed through a screen with an opening of 108 µm to give film coating solution (6).

### (6) Preparation of film-coated tablet comprising tartrate of the present compound

Film-coated tablets comprising tartrate of the present compound were prepared according to the conditions described in Table 10-6. The uncoated tablets for film-coating were placed in a coating machine (produced by FREUND CORPORATION, AQC-80 model), and heated under the conditions in Table 10-6 while the coating pan was rotated slightly until the exhaust temperature reached 50°C. Subsequently, while rotating the coating pan at 10 rpm, the film coating solution (6) was sprayed under the conditions in Table 10-6 to film-coat the tablets until each coating amount reached about 7.5 mg. Then, the film-coated tablets were dried until the exhaust temperature reached 60°C while the coating pan was rotated intermittently at 4.0 rpm. After drying, Polishing Wax 105 was added to the tablets to prepare film-coated tablets.

| Table 10-6. Preparation condition (Example 10, film-coated tablet) | |
|---|---|
| Item | Preparation condition |
| Number of spray guns | 2 |
| Spray air flow rate | 130 NL/MPa |
| Pattern air flow rate | 60 NL/MPa |
| Cylinder height | 2 memories |
| Static pressure inside pan | -100 Pa |
| Charge air temperature | 75°C |
| Charge air volume | 8.0 m³/min |
| Spray speed | about 80 g/min |

### Example 11: Tablet comprising tartrate of the present compound (11)

### A. Composition of uncoated tablet comprising tartrate of the present compound

Granules, mixed powders and uncoated tablets having the compositions shown in Table 11-1 were prepared.

| Table 11-1. Composition (Example 11) | | | |
|---|---|---|---|
| | Ingredient | Amount | |
| | | (mg) | (wt%) |
| Granule ingredient | tartrate of the present compound | 25.0 | 27.8 |
| | D-mannitol | 37.1 | 41.2 |
| | partially pregelatinized starch | 18.0 | 20.0 |
| | croscarmellose sodium | 1.8 | 2.0 |
| | polyvinyl alcohol | 4.5 | 5.0 |
| | Subtotal of granule | 86.4 | 96.0 |
| Mixture ingredient | sodium stearyl fumarate | 3.6 | 4.0 |
| | Subtotal of mixed powder and uncoated tablet | 90.0 | 100.0 |

### B. Manufacturing process

### (1) Preparation of 5 wt% polyvinyl alcohol solution (1)

Approximately 500 g of purified water was placed in a SUS beaker, and 100 g of EG-05P was gradually added to the beaker and the mixture was dispersed. Approximately 1500 g of purified water was heated to 80°C and added to the dispersed solution, and the mixture was dissolved. After leaving the solution to stand overnight, it was stirred again and the weight of the purified water that had evaporated was replenished to prepare 5 wt % polyvinyl alcohol solution (1) of 2000 g in total.

### (2) Preparation of granule comprising tartrate of the present compound

Granules comprising tartrate of the present compound were prepared using the ingredients and their amounts listed in Table 11-2 and under the preparation conditions listed in Table 11-3. First, tartrate of the present compound, D-mannitol, partially pregelatinized starch, and croscarmellose sodium in the amounts shown in Table 11-2 were charged into a tumbling fluid bed granulator dryer system (produced by POWREX CORPORATION, Multiplex MP-01 model) and mixed while fluidizing until the exhaust temperature reached 40°C. Subsequently, 5 wt% polyvinyl alcohol solution (1) in the amount shown in Table 11-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 11-2. Preparation amount (Example 11, granules) | | |
|---|---|---|
| | Ingredient | Amount (g) |
| Granule ingredient | tartrate of the present compound | 200.0 |
| | D-mannitol | 296.4 |
| | partially pregelatinized starch | 143.9 |
| | croscarmellose sodium | 14.4 |
| | 5 wt% polyvinyl alcohol solution (1) | 719.5 |

| Table 11-3. Preparation condition (Example 11, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 73 - 80°C |
| Charge air volume | 0.7 - 0.8 m³/min |
| Spray speed | about 13 g/min |

### (3) Preparation of mixed powder comprising tartrate of the present compound

Mixed powder comprising tartrate of the present compound was prepared in the preparation amounts shown in Table 11-4. Granules comprising tartrate of the present compound in the amount shown in Table 11-4 were placed in a 10 L V-shaped mixer (Tsutsui Scientific Instruments Co., Ltd., S-5 model), and then sodium stearyl fumarate in the amount shown in Table 11-4 was added thereto, and the contents were mixed in the mixer for 10 minutes at 40 rpm.

| Table 11-4. Preparation amount (Example 11, mixed powder) | | |
|---|---|---|
| | Ingredient | Amount (g) |
| Mixed ingredients | granules comprising tartrate of the present compound | 622.98 |
| | sodium stearyl fumarate | 25.96 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 11-5 so that each tablet comprises 25 mg of tartrate of the present compound.

| Table 11-5. Preparation condition (Example 11, uncoated tablet) | |
|---|---|
| Item | Preparation condition |
| Punch shape | Diameter: 6.0 mm, R: 8.0 mm |
| Turntable rotation speed | 20 rpm |
| Main pressure | 3.2 kN |

### Example 12: Tablet comprising tartrate of the present compound (12)

### A. Composition of uncoated tablet comprising tartrate of the present compound

Granules, mixed powders and uncoated tablets having the compositions shown in Table 12-1 were prepared.

| Table 12-1. Composition (Example 12) | | | |
|---|---|---|---|
| | Ingredient | Amount | |
| | | (mg) | (wt%) |
| Granule ingredient | tartrate of the present compound | 25.0 | 27.8 |
| | D-mannitol | 35.3 | 39.2 |
| | partially pregelatinized starch | 18.0 | 20.0 |
| | crospovidone | 3.6 | 4.0 |
| | hydroxypropyl cellulose | 4.5 | 5.0 |
| | Subtotal of granule | 86.4 | 96.0 |
| Mixture | sodium stearyl fumarate | 3.6 | 4.0 |
| ingredient | Subtotal of mixed powder and uncoated tablet | 90.0 | 100.0 |

### B. Manufacturing process

### (1) Preparation of 5 wt% hydroxypropyl cellulose solution (11)

2375 g of purified water was placed in a SUS beaker, and 125 g of HPC-L was gradually added to the beaker while stirring to dissolve the mixture. After leaving the solution to stand overnight, it was stirred again to prepare a 5 wt% hydroxypropyl cellulose solution (11).

### (2) Preparation of granule comprising tartrate of the present compound

Granules comprising tartrate of the present compound were prepared using the ingredients and their amounts listed in Table 12-2 and under the preparation conditions listed in Table 12-3. First, tartrate of the present compound, D-mannitol, partially pregelatinized starch, and crospovidone in the amounts shown in Table 12-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (11) in the amount shown in Table 12-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 12-2. Preparation amount (Example 12, granules) | | |
|---|---|---|
| | Ingredient | Amount (g) |
| Granule ingredient | tartrate of the present compound | 200.0 |
| | D-mannitol | 282.0 |
| | partially pregelatinized starch | 143.9 |
| | crospovidone | 28.8 |
| | 5 wt% hydroxypropyl cellulose solution (11) | 719.5 |

| Table 12-3. Preparation condition (Example 12, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 73°C |
| Charge air volume | 0.7 m³/min |
| Spray speed | about 13 g/min |

### (3) Preparation of mixed powder comprising tartrate of the present compound

Mixed powder comprising tartrate of the present compound was prepared in the preparation amounts shown in Table 12-4. Granules comprising tartrate of the present compound in the amount shown in Table 12-4 were placed in a 10 L V-shaped mixer (Tsutsui Scientific Instruments Co., Ltd., S-5 model), and then sodium stearyl fumarate in the amount shown in Table 12-4 was added thereto, and the contents were mixed in the mixer for 10 minutes at 40 rpm.

| ITable 12-4. Preparation amount (Example 12, mixed powder) | | |
|---|---|---|
| | Ingredient | Amount (g) |
| Mixed ingredients | granules comprising tartrate of the present compound | 625.09 |
| | sodium stearyl fumarate | 26.05 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 12-5 so that each tablet comprises 25 mg of tartrate of the present compound.

| Table 12-5. Preparation condition (Example 12, uncoated tablet) | |
|---|---|
| Item | Preparation condition |
| Punch shape | Diameter: 6.0 mm, R: 8.0 mm |
| Turntable rotation speed | 20 rpm |
| Main pressure | 3.7 kN |

### Example 13: Tablet comprising tartrate of the present compound (13)

### A. Composition of uncoated tablet comprising tartrate of the present compound

Granules, mixed powders and uncoated tablets having the compositions shown in Table 13-1 were prepared.

| Table 13-1. Composition (Example 13) | | | |
|---|---|---|---|
| | Ingredient | Amount | |
| | | (mg) | (wt%) |
| Granule ingredient | tartrate of the present compound | 25.0 | 27.8 |
| | D-mannitol | 37.1 | 41.2 |
| | partially pregelatinized starch | 18.0 | 20.0 |
| | sodium starch glycolate | 1.8 | 2.0 |
| | hydroxypropyl cellulose | 4.5 | 5.0 |
| | Subtotal of granule | 86.4 | 96.0 |
| Mixture ingredient | sodium stearyl fumarate | 3.6 | 4.0 |
| | Subtotal of mixed powder and uncoated tablet | 90.0 | 100.0 |

### B. Manufacturing process

### (1) Preparation of 5 wt% hydroxypropyl cellulose solution (12)

2375 g of purified water was placed in a SUS beaker, and 125 g of HPC-L was gradually added to the beaker while stirring to dissolve the mixture. After leaving the solution to stand overnight, it was stirred again to prepare a 5 wt% hydroxypropyl cellulose solution (12).

### (2) Preparation of granule comprising tartrate of the present compound

Granules comprising tartrate of the present compound were prepared using the ingredients and their amounts listed in Table 13-2 and under the preparation conditions listed in Table 13-3. First, tartrate of the present compound, D-mannitol, partially pregelatinized starch, and sodium starch glycolate in the amounts shown in Table 13-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (12) in the amount shown in Table 13-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 13-2. Preparation amount (Example 13, granules) | | |
|---|---|---|
| | Ingredient | Amount (g) |
| Granule ingredient | tartrate of the present compound | 200.0 |
| | D-mannitol | 296.4 |
| | partially pregelatinized starch | 143.9 |
| | sodium starch glycolate | 14.4 |
| | 5 wt% hydroxypropyl cellulose solution (12) | 719.5 |

| Table 13-3. Preparation condition (Example 13, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 73°C |
| Charge air volume | 0.7 m³/min |
| Spray speed | about 13 g/min |

### (3) Preparation of mixed powder comprising tartrate of the present compound

Mixed powder comprising tartrate of the present compound was prepared in the preparation amounts shown in Table 13-4. Granules comprising tartrate of the present compound in the amount shown in Table 13-4 were placed in a 10 L V-shaped mixer (Tsutsui Scientific Instruments Co., Ltd., S-5 model), and then sodium stearyl fumarate in the amount shown in Table 13-4 was added thereto, and the contents were mixed in the mixer for 10 minutes at 40 rpm.

| Table 13-4. Preparation amount (Example 13, mixed powder) | | |
|---|---|---|
| | Ingredient | Amount (g) |
| Mixed ingredients | granules comprising tartrate of the present compound | 634. 41 |
| | sodium stearyl fumarate | 26.43 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 13-5 so that each tablet comprises 25 mg of tartrate of the present compound.

| Table 13-5. Preparation condition (Example 13, uncoated tablet) | |
|---|---|
| Item | Preparation condition |
| Punch shape | Diameter: 6.0 mm, R: 8.0 mm |
| Turntable rotation speed | 20 rpm |
| Main pressure | 3.5 kN |

### Example 14: Tablet comprising tartrate of the present compound (14)

In the present example, the following partially pregelatinized starch was used:
STARCH1500^{™}: Colorcon

### A. Composition of uncoated tablet comprising tartrate of the present compound

Granules, mixed powders and uncoated tablets having the compositions shown in Table 14-1 were prepared.

| Table 14-1. Composition (Example 14) | | | |
|---|---|---|---|
| | Ingredient | Amount | |
| | | (mg) | (wt%) |
| Granule ingredient | tartrate of the present compound | 25.0 | 27.8 |
| | D-mannitol | 37.1 | 41.2 |
| | partially pregelatinized starch | 18.0 | 20.0 |
| | croscarmellose sodium | 1.8 | 2.0 |
| | hydroxypropyl cellulose | 4.5 | 5.0 |
| | Subtotal of granule | 86.4 | 96.0 |
| Mixture ingredient | sodium stearyl fumarate | 3.6 | 4.0 |
| | Subtotal of mixed powder and uncoated tablet | 90.0 | 100.0 |

### B. Manufacturing process

### (1) Preparation of 5 wt% hydroxypropyl cellulose solution (13)

2375 g of purified water was placed in a SUS beaker, and 125 g of HPC-L was gradually added to the beaker while stirring to dissolve the mixture. After leaving the solution to stand overnight, it was stirred again to prepare a 5 wt% hydroxypropyl cellulose solution (13).

### (2) Preparation of granule comprising tartrate of the present compound

Granules comprising tartrate of the present compound were prepared using the ingredients and their amounts listed in Table 14-2 and under the preparation conditions listed in Table 14-3. First, tartrate of the present compound, D-mannitol, partially pregelatinized starch, and croscarmellose sodium in the amounts shown in Table 14-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (13) in the amount shown in Table 14-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 14-2. Preparation amount (Example 14, granules) | | |
|---|---|---|
| | Ingredient | Amount (g) |
| Granule ingredient | tartrate of the present compound | 200.0 |
| | D-mannitol | 296.4 |
| | partially pregelatinized starch | 143.9 |
| | croscarmellose sodium | 14.4 |
| | 5 wt% hydroxypropyl cellulose solution (13) | 719.5 |

| Table 14-3. Preparation condition (Example 14, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 73°C |
| Charge air volume | 0.7 m³/min |
| Spray speed | about 13 g/min |

### (3) Preparation of mixed powder comprising tartrate of the present compound

Mixed powder comprising tartrate of the present compound was prepared in the preparation amounts shown in Table 14-4. Granules comprising tartrate of the present compound in the amount shown in Table 14-4 were placed in a 10 L V-shaped mixer (Tsutsui Scientific Instruments Co., Ltd., S-5 model), and then sodium stearyl fumarate in the amount shown in Table 14-4 was added thereto, and the contents were mixed in the mixer for 10 minutes at 40 rpm.

| Table 14-4. Preparation amount (Example 14, mixed powder) | | |
|---|---|---|
| | Ingredient | Amount (g) |
| Mixed ingredients | granules comprising tartrate of the present compound | 622.85 |
| | sodium stearyl fumarate | 25.95 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 14-5 so that each tablet comprises 25 mg of tartrate of the present compound.

| Table 14-5. Preparation condition (Example 14, uncoated tablet) | |
|---|---|
| Item | Preparation condition |
| Punch shape | Diameter: 6.0 mm, R: 8.0 mm |
| Turntable rotation speed | 20 rpm |
| Main pressure | 3.5 kN |

### Comparative example 1: Tablet comprising tartrate of the present compound (15)

### A. Composition of uncoated tablet comprising tartrate of the present compound

Granules, mixed powders and uncoated tablets having the compositions shown in Table 15-1 were prepared.

| Table 15-1. Composition (Comparative example 1) | | | |
|---|---|---|---|
| | Ingredient | Amount | |
| | | (mg) | (wt%) |
| Granule ingredient | tartrate of the present compound | 50.0 | 20.0 |
| | D-mannitol | 135.0 | 54.0 |
| | partially pregelatinized starch | 50.0 | 20.0 |
| | croscarmellose sodium | 5.0 | 2.0 |
| | hydroxypropyl cellulose | 5.0 | 2.0 |
| | Subtotal of granule | 245.0 | 98.0 |
| Mixture ingredient | sodium stearyl fumarate | 5.0 | 2.0 |
| | Subtotal of mixed powder and uncoated tablet | 250.0 | 100.0 |

### B. Manufacturing process

### (1) Preparation of 5 wt% hydroxypropyl cellulose solution (14)

684 g of purified water was placed in a SUS beaker, and 36 g of HPC-L was gradually added to the beaker while stirring to dissolve the mixture. After leaving the solution to stand overnight, it was stirred again to prepare a 5 wt% hydroxypropyl cellulose solution (14).

### (2) Preparation of granule comprising tartrate of the present compound

Granules comprising tartrate of the present compound were prepared using the ingredients and their amounts listed in Table 15-2 and under the preparation conditions listed in Table 15-3. First, tartrate of the present compound, D-mannitol, partially pregelatinized starch, and croscarmellose sodium in the amounts shown in Table 15-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (14) in the amount shown in Table 15-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 15-2. Preparation amount (Comparative example 1, granules) | | |
|---|---|---|
| | Ingredient | Amount (g) |
| | tartrate of the present compound | 140 |
| | D-mannitol | 385 |
| Granule ingredient | partially pregelatinized starch | 140 |
| | croscarmellose sodium | 14 |
| | 5 wt% hydroxypropyl cellulose solution (14) | 280.0 |

| Table 15-3. Preparation condition (Comparative example 1, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 70 - 72°C |
| Charge air volume | 0.6 - 0.7 m³/min |
| Spray speed | about 13 g/min |

### (3) Preparation of mixed powder comprising tartrate of the present compound

Mixed powder comprising tartrate of the present compound was prepared in the preparation amounts shown in Table 15-4. Granules comprising tartrate of the present compound in the amount shown in Table 15-4 were placed in a plastic bag, and then sodium stearyl fumarate in the amount shown in Table 15-4 was added thereto, and the contents of the bag were mixed (50 rotations vertically, 50 rotations horizontally, a total of 100 rotations).

| Table 15-4. Preparation amount (Comparative example 1, mixed powder) | | |
|---|---|---|
| | Ingredient | Amount (g) |
| Mixed ingredients | granules comprising tartrate of the present compound | 313.56 |
| | sodium stearyl fumarate | 6.40 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 15-5 so that each tablet comprises 50 mg of tartrate of the present compound.

| Table 15-5. Preparation condition (Comparative example 1, uncoated tablet) | |
|---|---|
| Item | Preparation condition |
| Punch shape | Diameter: 9.0 mm, R: 13.0 mm |
| Turntable rotation speed | 20 rpm |
| Main pressure | 4.3 kN |

### Comparative example 2: Tablet comprising tartrate of the present compound (16)

### A. Composition of uncoated tablet comprising tartrate of the present compound

Granules, mixed powders and uncoated tablets having the compositions shown in Table 16-1 were prepared.

| Table 16-1. Composition (Comparative example 2) | | | |
|---|---|---|---|
| | Ingredient | Amount | |
| | | (mg) | (wt%) |
| Granule ingredient | tartrate of the present compound | 50.0 | 20.0 |
| | D-mannitol | 137.5 | 55.0 |
| | partially pregelatinized starch | 50.0 | 20.0 |
| | croscarmellose sodium | 5.0 | 2.0 |
| | hydroxypropyl cellulose | 5.0 | 2.0 |
| | Subtotal of granule | 247.5 | 99.0 |
| Mixture ingredient | magnesium stearate | 2.5 | 1.0 |
| | Subtotal of mixed powder and uncoated tablet | 250.0 | 100.0 |

### B. Manufacturing process

### (1) Preparation of 5 wt% hydroxypropyl cellulose solution (15)

684 g of purified water was placed in a SUS beaker, and 36 g of HPC-L was gradually added to the beaker while stirring to dissolve the mixture. After leaving the solution to stand overnight, it was stirred again to prepare a 5 wt% hydroxypropyl cellulose solution (15).

### (2) Preparation of granule comprising tartrate of the present compound

Granules comprising tartrate of the present compound were prepared using the ingredients and their amounts listed in Table 16-2 and under the preparation conditions listed in Table 16-3. First, tartrate of the present compound, D-mannitol, partially pregelatinized starch, and croscarmellose sodium in the amounts shown in Table 16-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hydroxypropyl cellulose solution (15) in the amount shown in Table 16-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 16-2. Preparation amount (Comparative example 2, granules) | | |
|---|---|---|
| | Ingredient | Amount (g) |
| Granule ingredient | tartrate of the present compound | 140 |
| | D-mannitol | 385 |
| | partially pregelatinized starch | 140 |
| | croscarmellose sodium | 14 |
| | 5 wt% hydroxypropyl cellulose solution (15) | 280.0 |

| Table 16-3. Preparation condition (Comparative example 2, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 67°C |
| Charge air volume | 0.5 - 0.7 m³/min |
| Spray speed | about 8 g/min |

### (3) Preparation of mixed powder comprising tartrate of the present compound

Mixed powder comprising tartrate of the present compound was prepared in the preparation amounts shown in Table 16-4. Granules comprising tartrate of the present compound in the amount shown in Table 16-4 were placed in a plastic bag, and then sodium stearyl fumarate in the amount shown in Table 16-4 was added thereto, and the contents of the bag were mixed (50 rotations vertically, 50 rotations horizontally, a total of 100 rotations).

| Table 16-4. Preparation amount (Comparative example 2, mixed powder) | | |
|---|---|---|
| | Ingredient | Amount (g) |
| Mixed ingredients | granules comprising tartrate of the present compound | 304.33 |
| | magnesium stearate | 3.07 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 16-5 so that each tablet comprises 50 mg of tartrate of the present compound.

| Table 16-5. Preparation condition (Comparative example 2, uncoated tablet) | |
|---|---|
| Item | Preparation condition |
| Punch shape | Diameter: 9.0 mm, R: 13.0 mm |
| Turntable rotation speed | 20 rpm |
| Main pressure | 6.0 kN |

### Comparative example 3: Tablet comprising tartrate of the present compound (17)

### A. Composition of uncoated tablet comprising tartrate of the present compound

Granules, mixed powders and uncoated tablets having the compositions shown in Table 17-1 were prepared.

| Table 17-1. Composition (Comparative example 3) | | | |
|---|---|---|---|
| | Ingredient | Amount | |
| | | (mg) | (wt%) |
| Granule ingredient | tartrate of the present compound | 25.0 | 27.8 |
| | D-mannitol | 37.1 | 41.2 |
| | partially pregelatinized starch | 18.0 | 20.0 |
| | croscarmellose sodium | 1.8 | 2.0 |
| | hypromellose | 4.5 | 5.0 |
| | Subtotal of granule | 86.4 | 96.0 |
| Mixture ingredient | sodium stearyl fumarate | 3.6 | 4.0 |
| | Subtotal of mixed powder and uncoated tablet | 90.0 | 100.0 |

### B. Manufacturing process

### (1) Preparation of 5 wt% hypromellose solution (1)

1900 g of purified water was placed in a SUS beaker, and 100 g of HPC-L was gradually added to the beaker while stirring to dissolve the mixture. After leaving the solution to stand overnight, it was stirred again to prepare a 5 wt% hypromellose solution (1).

### (2) Preparation of granule comprising tartrate of the present compound

Granules comprising tartrate of the present compound were prepared using the ingredients and their amounts listed in Table 17-2 and under the preparation conditions listed in Table 17-3. First, tartrate of the present compound, D-mannitol, partially pregelatinized starch, and croscarmellose sodium in the amounts shown in Table 17-2 were charged into a tumbling fluid bed granulator dryer system (produced by POWREX CORPORATION, Multiplex MP-01 model) and mixed while fluidizing until the exhaust temperature reached 40°C. Subsequently, 5 wt% hypromellose solution (1) in the amount shown in Table 17-2 was sprayed onto the mixture while fluidizing. Then, the mixture was dried while being fluidized until the exhaust temperature reached 45°C, and then removed from the container. The removed granules were passed through a sieve with 710 µm openings to obtain granules comprising tartrate salt of the present compound.

| Table 17-2. Preparation amount (Comparative example 3, granules) | | |
|---|---|---|
| | Ingredient | Amount (g) |
| Granule ingredient | tartrate of the present compound | 200 |
| | D-mannitol | 296.4 |
| | partially pregelatinized starch | 143.9 |
| | croscarmellose sodium | 14.4 |
| | 5 wt% hypromellose solution (1) | 719.5 |

| Table 17-3. Preparation condition (Comparative example 3, granules) | |
|---|---|
| Item | Preparation condition |
| Container used | standard container |
| Gun bore | 1.0 mm |
| Gun height | low |
| Spray type | top-spray |
| Spray pressure | 0.12 MPa |
| Charge air temperature | 73°C |
| Charge air volume | 0.6 - 0.7 m³/min |
| Spray speed | about 13 g/min |

### (3) Preparation of mixed powder comprising tartrate of the present compound

Mixed powder comprising tartrate of the present compound was prepared in the preparation amounts shown in Table 17-4. Granules comprising tartrate of the present compound in the amount shown in Table 17-4 were placed in a 10 L V-shaped mixer (Tsutsui Scientific Instruments Co., Ltd., S-5 model), and then sodium stearyl fumarate in the amount shown in Table 17-4 was added thereto, and the contents were mixed in the mixer for 10 minutes at 40 rpm.

| Table 17-4. Preparation amount (Comparative example 3, mixed powder) | | |
|---|---|---|
| | Ingredient | Amount (g) |
| Mixed ingredients | granules comprising tartrate of the present compound | 615.46 |
| | sodium stearyl fumarate | 25.64 |

### (4) Preparation of uncoated tablet comprising tartrate of the present compound

The above mixed powder was compressed into uncoated tablets using a rotary tableting machine (produced by KIKUSUI SEISAKUSHO LTD, VELC) under the condition shown in Table 17-5 so that each tablet comprises 25 mg of tartrate of the present compound.

| Table 17-5. Preparation condition (Comparative example 3, uncoated tablet) | |
|---|---|
| Item | Preparation condition |
| Punch shape | Diameter: 6.0 mm, R: 8.0 mm |
| Turntable rotation speed | 20 rpm |
| Main pressure | 4.0 kN |

The quality of the formulations prepared by the above processes was evaluated by the following method.

### Test 1: Evaluation of tabletability, and measurement of shape, hardness, and friability of uncoated tablets

As for Examples 1 to 14 and Comparative examples 1 and 2, the tabletability was evaluated, and the thickness, hardness, and friability of their uncoated tablets were measured. The thickness of the uncoated tablets was measured using a special Digimatic Thickness Gauge 547-301, and the hardness was measured using a tablet-breaking hardness tester TH-203MP manufactured by Toyama Sangyo, and the average value for 5 or 10 tablets was recorded. In addition, the tablet friability test was performed according to the test method described in the Japanese Pharmacopoeia, using a FRIABILATOR TFT-1200 manufactured by Toyama Sangyo. The measurement results are shown in Table 18.

| Table 18. Measurement results of shape, hardness, and friability of uncoated tablets | | | | | |
|---|---|---|---|---|---|
| | tabletability | Shape of uncoated tablet (mm) | | Hardness of uncoated tablet | Result of friability test |
| | | Tablet diameter | Thickness | | friability |
| Example 1 | - | 8.0 | 3.65 | 76 N | 0.03% |
| Example 2 | ± | 9.0 | 4.29 | 73 N | 0.00% |
| Example 3 | - | 6.0 | 3.16 | 62 N | 0.12% |
| Example 4 | - | 8.0 | 3.64 | 77 N | 0.16% |
| Example 5 | ± | 14.0 x 6.5 | 4.65 | 114 N | 0.15% |
| Example 6 | ± | 8.0 | 3.66 | 79 N | 0.09% |
| Example 7 | ± | 8.0 | 3.66 | 60 N | 0.15% |
| Example 8 | - | 6.0 | 3.12 | 59 N | 0.14% |
| Example 9 | ± | 14.0 x 6.5 | 4.47 | 107 N | 0.27% |
| Example 10 | - | 16.0 x 6.5 | 5.16 | 133 N | 0.10% |
| Example 11 | + | 6.0 | 3.21 | 57 N | 0.17% |
| Example 12 | + | 6.0 | 3.21 | 51 N | 0.22% |
| Example 13 | + | 6.0 | 3.18 | 54 N | 0.19% |
| Example 14 | + | 6.0 | 3.18 | 56 N | 0.17% |
| Comparative example 1 | + | 9.0 | 4.27 | 40 N | 0.14% |
| Comparative example 2 | ++ | 9.0 | 4.22 | 53 N | |
| [Tabletability] | | | | | |
| -: There were no problems with tableting, and continuous tableting was possible. | | | | | |
| ±: The tablets and punches became slightly cloudy, but continuous tableting was possible. | | | | | |
| +: Sticking occurred in the punch, causing some of the tablet surface to break off, making continuous tableting difficult. | | | | | |
| ++: Sticking occurred in the punch, and die friction was also observed, making continuous tableting difficult. | | | | | |
| [Friability] | | | | | |
| When the friability was negative, the friability was recorded as 0%. | | | | | |

As can be seen from Table 18, Examples 1 to 14 exhibited good tabletability, hardness, and friability. As for Comparative Example 1 comprising 2% binder and 2% lubricant, sticking occurred, the tabletability was poor, and the hardness of the uncoated tablets was also low. In addition, as for Comparative example 2 comprising 1% magnesium stearate as a lubricant, sticking occurred, die friction was observed, and the hardness was even lower than in Comparative Example 1, making continuous tableting difficult. It has been found that by using 4% or more of a binder and 2% or more of a lubricant, tablets exhibiting significantly excellent tabletability and high hardness could be produced.

### Test 2: Dissolution test

As for Examples 1 to 14 and Comparative example 3, dissolution tests were carried out under the following conditions in accordance with the Japanese Pharmacopoeia, General Test Method, Dissolution Test Method.

### <Dissolution test condition>

Method: Dissolution Test of the Japanese Pharmacopoeia (Paddle Method)
Rotation speed: 50 rpm
Test medium volume: 900 mL
Medium temperature: 37°C±0.5°C
Test medium: 0.1 N HCl

### <Preparation of sample solution>

A tablet is placed in a test vessel containing 900 mL of test medium, and 5, 10, and 15 minutes after the start of the test, 10 mL of the solution for test is sampled using a 10 mL syringe and slowly filtered using a membrane filter with a pore size of 0.45 µm or less. The first 2 mL or more of the filtrate is removed, and the remaining filtrate is used as a sample solution.

### <Preparation of standard solution>

50.00 mg of tartrate salt of the present compound was

weighed into a 300 mL volumetric flask and dissolved in the dissolution test medium. Subsequently, the solution was weighed to 10 mL in a 30 mL volumetric flask and dissolved in the dissolution test medium. The solution was used as a mother liquid of the standard solution.

The mother liquor was diluted according to the volume of each sample to prepare each standard solution.

### <High performance liquid chromatography, Analysis condition 1>

Analysis condition 1 was used in the tests of Example 1 and Example 2.
High performance liquid chromatography machine: Shimadzu UFLC-XR
Detective wavelength: 260 nm
Column: Waters, Xselect CSH C18 150 mm x 4.6 mm I.D. particle size 3.5 µm
Mobile phase: a mixture of water/methanol/perchloric acid (1000/1000/1)
Analytical time: 5 min
Flow rate: 0.8 mL/min
Column temperature: 40°C
Injection volume: 5 µL
Temperature of sample cooler: 5°C
Washing solution for syringe: a mixture of methanol/water (1/1)

### <High performance liquid chromatography, Analysis condition 2>

Analysis condition 2 was used in the tests of Examples 3 - 14 and Reference examples 3.
High performance liquid chromatography machine: Shimadzu UFLC-XR
Detective wavelength: 260 nm
Column: Waters, Xselect CSH C18 150 mm x 4.6 mm I.D particle size 3.5 µm
Mobile phase: a mixture of water/methanol/ perchloric acid (1000/1000/3)
Analytical time: 5 min
Flow rate: 0.8 mL/min
Column temperature: 40°C
Injection volume: 5 µL
Temperature of sample cooler: 25°C
Washing solution for syringe: a mixture of methanol/water (1/1)

The results of dissolution test are shown in Table 19.

| Table 19. Result of dissolution test | | | | |
|---|---|---|---|---|
| | Tablet | Dissolution rate at 5 min | Dissolution rate at 10 min | Dissolution rate at 15 min |
| Example 1 | uncoated tablet | 58.9% | 96.5% | 98.1% |
| | FC tablet | 40.2% | 91.9% | 97.1% |
| Example | uncoated | 55.8% | 95.2% | 100.9% |

| | | | | |
|---|---|---|---|---|
| 2 | tablet | | | |
| Example 3 | uncoated tablet | 71.2% | 98.1% | 98.6% |
| | FC tablet | 41.7% | 98.0% | 99.7% |
| Example 4 | uncoated tablet | 38.9% | 97.9% | 101.5% |
| | FC tablet | 26.8% | 84.3% | 97.0% |
| Example 5 | uncoated tablet | 48.1% | 90.0% | 98.4% |
| | FC tablet | 26.5% | 85.8% | 98.0% |
| Example 6 | uncoated tablet | 43.0% | 88.4% | 101.9% |
| Example 7 | uncoated tablet | 79.1% | 99.2% | 99.5% |
| Example 8 | uncoated tablet | 69.1% | 100.0% | 100.5% |
| | FC tablet | 50.9% | 98.9% | 99.6% |
| Example 9 | uncoated tablet | 42.4% | 90.9% | 98.3% |
| Example 10 | uncoated tablet | 44.4% | 87.6% | 99.7% |
| | FC tablet | 21.2% | 74.5% | 97.0% |
| Example 11 | uncoated tablet | 35.2% | 65.1% | 86.1% |
| Example 12 | uncoated tablet | 76.3% | 99.2% | 99.5% |
| Example 13 | uncoated tablet | 64.3% | 99.7% | 100.5% |
| Example 14 | uncoated tablet | 73.7% | 101.1% | 102.3% |
| Reference example 3 | uncoated tablet | 28.2% | 53.2% | 72.5% |

As shown in Table 19, the uncoated tablets and FC tablets of the present invention exhibited rapid dissolution, indicating that high absorption *in vivo* can be expected. Although a slight difference was observed in the initial dissolution profiles depending on the presence or absence of film coating, there was almost no difference in the dissolution rate at 15 minutes, indicating that the film coating did not affect the dissolution properties. As the results show, tablets were able to be produced that exhibited good dissolution properties, *i.e.,* rapid dissolution, despite the use of relatively large amounts of binder and lubricant.

### Test 3: Storage stability test (test of purity)

The temperature and humidity of the constant temperature and humidity chamber were set at 40°C/75% RH or 50°C/85% RH. The uncoated tablets prepared in Examples 1-14 were placed in HDPE bottles or amber screw-cap test tubes (glass) and stored in an open condition in the chamber for a predetermined period. Thereafter, a test of purity was performed, and the amounts of related substances were evaluated.

### Test condition 1

Test condition 1 was used in the tests of Example 1 and Example 2.
Detector: ultraviolet absorption photometer (measurement wavelength: 220 nm)
Column: Waters, Xselect CSH C18 150 mm x 4.6 mm I.D. particle size 3.5 µm
Column temperature: constant temperature around 40°C Mobile phase A: a mixture of water/ perchloric acid (10000:1) Mobile phase B: methanol
Elution method: gradient method
Flow rate: 0.8 mL/min
Area measurement: for 50 minutes after injection
Temperature of sample cooler: 5°C
Dissolving solvent: a mixture of water/methanol/perchloric acid (1000/1000/1)
Washing solution for syringe: a mixture of methanol/water (1/1)

### Test condition 2

Test condition 2 was used in the tests of Examples 3 - 14.
Detector: ultraviolet absorption photometer (measurement wavelength: 220 nm)
Column: Waters, Xselect CSH C18 150 mm x 4.6 mm I.D. particle size 3.5 µm
Column temperature: constant temperature around 40°C
Mobile phase A: a mixture of water/ perchloric acid (10000:1)
Mobile phase B: methanol
Elution method: gradient method
Flow rate: 0.8 mL/min
Area measurement: for 60 minutes after injection
Temperature of sample cooler: 25°C
Dissolving solvent: a mixture of water/methanol/perchloric acid (1000/1000/3)
Washing solution for syringe: a mixture of methanol/water (1/1)

Based on the peak area values obtained by liquid chromatography, the amounts of related substances relative to the present compound were calculated. Among these, the results for the total amount of the detected related substances are shown in Tables 20-1 and 20-2.

| Table 20-1. Stability test results of uncoated tablets (40°C/75%RH) | | | | |
|---|---|---|---|---|
| | Amount of related substances | | | |
| | Initial | 1M | 3M | 6M |
| Example 1 | 1.01% | 1.00% | 1.02% | 1.07% |
| Example 2 | 1.02% | 1.03% | 1. 04% | 1.08% |
| Example 3 | 0.80% | 0.84% | 0.84% | 0.97% |
| Example 4 | 0.77% | 0.83% | 0.82% | 0.96% |
| Example 5 | 0.79% | 0.82% | 0.82% | 0.93% |
| Example 6 | 0.79% | 0.81% | 0.84% | 0.94% |
| Example 7 | 0.79% | 0.85% | 0.83% | 0.95% |
| Example 8 | 0.79% | 0.84% | 0.84% | 0.95% |
| Example 9 | 0.79% | 0.80% | 0.90% | |
| Example 10 | 0.86% | 0.82% | 0.82% | 0.84% |
| Example 11 | 0.80% | 0.82% | 0.90% | |
| Example 12 | 0.78% | 0.84% | 0.90% | |
| Example 13 | 0.80% | 0.80% | 0.90% | |
| Example 14 | 0.79% | 0.83% | 0.91% | |

| Table 20-2. Stability test results of uncoated tablets (50°C/85%RH) | | | |
|---|---|---|---|
| | Amount of related substances | | |
| | Initial | 2w | 4w |
| Example 1 | 1.01% | 1.01% | 1.02% |
| Example 2 | 1.02% | 1.04% | 1.07% |
| Example 3 | 0.80% | 0.78% | 0.81% |
| Example 4 | 0.77% | 0.79% | 0.84% |
| Example 5 | 0.79% | 0.78% | 0.82% |
| Example 6 | 0.79% | 0.85% | 0.84% |
| Example 7 | 0.79% | 0.83% | 0.85% |
| Example 8 | 0.79% | 0.77% | 0.82% |
| Example 9 | 0.79% | 0.80% | 0.80% |
| Example 10 | 0.86% | 0.82% | 0.82% |
| Example 11 | 0.80% | 0.82% | 0.82% |
| Example 12 | 0.78% | 0.81% | 0.80% |
| Example 13 | 0.80% | 0.79% | 0.77% |
| Example 14 | 0.79% | 0.80% | 0.80% |

As shown in Tables 20-1 and 20-2, the uncoated tablets of the present invention showed no increase in related substances during the storage stability test and therefore exhibited good storage stability.

### Test 4: Storage stability test (dissolution test)

The temperature and humidity of the constant temperature and humidity chamber were set at 40°C/75% RH. The uncoated tablets or FC tablets prepared in Examples 1-14 were placed in HDPE bottles and stored in an open condition in the chamber for a predetermined period. Thereafter, dissolution tests were carried out in accordance with the Japanese Pharmacopoeia, General Test Method, Dissolution Test Method, under the conditions described in Test 2.

The results of the dissolution test are shown in Tables 21-1 and 21-2.

| Table 21-1. Result of dissolution test (40°C/75%RH, 1M) | | | | |
|---|---|---|---|---|
| | Tablet | Dissolution rate at 5 min | Dissolution rate at 10 min | Dissolution rate at 15 min |
| Example 1 | uncoated tablet | 77.1% | 95.5% | 96.2% |
| | FC tablet | 63.0% | 96.8% | 97.5% |
| Example 2 | uncoated tablet | 68.9% | 91.5% | 99.9% |
| Example 3 | uncoated tablet | 89.7% | 99.8% | 100.0% |
| Example 4 | uncoated tablet | 77.7% | 99.1% | 99.8% |
| Example 5 | uncoated tablet | 79.2% | 98.7% | 99.8% |
| | FC tablet | 41.6% | 95.2% | 99.5% |
| Example 6 | uncoated tablet | 69.7% | 99.1% | 100.5% |
| Example 7 | uncoated tablet | 93.4% | 98.9% | 99.1% |
| Example 8 | uncoated tablet | 79.6% | 97.6% | 97.9% |
| Example 9 | uncoated tablet | 79.0% | 97.9% | 100.6% |
| Example 10 | uncoated tablet | 80.9% | 99.4% | 100.9% |
| | FC tablet | 48.4% | 93.1% | 99.7% |
| Example 12 | uncoated tablet | 95.2% | 99.4% | 99.4% |
| Example 13 | uncoated tablet | 87.6% | 100.3% | 100.6% |
| Example 14 | uncoated tablet | 77.0% | 95.1% | 99.4% |

| Table 21-2. Result of dissolution test (40°C/75%RH, 3M) | | | | |
|---|---|---|---|---|
| | Tablet | Dissolution rate at 5 min | Dissolution rate at 10 min | Dissolution rate at 15 min |
| Example 1 | uncoated tablet | 74.8% | 93.6% | 96.3% |
| | FC tablet | 55.9% | 95.0% | 96.6% |
| Example 2 | uncoated tablet | 72.6% | 95.4% | 100.2% |
| Example 3 | uncoated tablet | 89.6% | 99.3% | 99.4% |
| Example 4 | uncoated tablet | 77.6% | 98.4% | 99.8% |
| Example 5 | uncoated tablet | 80.3% | 98.0% | 98.9% |
| | FC tablet | 48.3% | 92.1% | 96.9% |
| Example 6 | uncoated tablet | 66% | 93% | 98% |
| Example 7 | uncoated tablet | 84% | 98% | 98% |
| Example 8 | uncoated tablet | 84.4% | 99.4% | 99.7% |
| Example 9 | uncoated tablet | 69.3% | 94.8% | 99.5% |
| Example 10 | uncoated tablet | 67.2% | 97.0% | 99.3% |
| | FC tablet | 42.4% | 88.3% | 97.2% |
| Example 11 | uncoated tablet | 41.3% | 72.5% | 92.2% |
| Example 12 | uncoated tablet | 97.6% | 100.9% | 100.9% |
| Example 13 | uncoated tablet | 87.7% | 102.0% | 102.5% |
| Example 14 | uncoated tablet | 84.1% | 101.7% | 101.9% |

As shown in Tables 21-1 and 21-2, the uncoated tablets or FC tablets of the present invention exhibited rapid dissolution, indicating that high absorption *in vivo* can be expected. Although a slight difference was observed in the initial dissolution profiles depending on the presence or absence of film coating, there was almost no difference in the dissolution rate at 15 minutes, indicating that the film coating did not affect the dissolution properties. As the results show, tablets were able to be produced that exhibited good dissolution properties, *i.e.,* rapid dissolution, despite the use of relatively large amounts of binder and lubricant.

As shown by the above results, it has been revealed that the formulation of the present invention has good dissolution properties and good storage stability, and furthermore, when provided as a tablet, it suppresses tableting problems.

As described above, the present disclosure has been illustrated using preferred embodiments; however, it is understood that the scope of the present invention should be construed only by the claims that follow. It is understood that the patents, patent applications, and other references cited herein are incorporated by reference into this specification in their entirety as if the contents were specifically set forth herein.

### INDUSTRIAL APPLICABILITY

According to the present disclosure, it is possible to provide an oral solid pharmaceutical formulation comprising (i) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide, or a pharmaceutically acceptable salt thereof, or a hydrate or solvate thereof, as an active ingredient, (ii) a binder, and (iii) a lubricant, wherein the binder and the lubricant are contained in high amounts, such that tableting problems are suppressed and excellent dissolution properties are achieved.

## Claims

1. An oral solid formulation comprising
(1) 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof,
(2) a binder in 4.0 - 6.0% (w/w) per 100% (w/w) of the formulation, and
(3) a lubricant in 2.0 - 10.0% (w/w) per 100% (w/w) of the formulation.

2. The oral solid formulation of claim 1, wherein the content of the binder is 5.0 - 6.0% (w/w) per 100% (w/w) of the formulation.

3. The oral solid formulation of claim 1 or 2, wherein the binder is a water-soluble polymer binder.

4. The oral solid formulation of claim 3, wherein the water-soluble polymer binder is hydroxypropyl cellulose or polyvinyl alcohol.

5. The oral solid formulation of claim 3, wherein the water-soluble polymer binder is hydroxypropyl cellulose.

6. The oral solid formulation of any one of claims 1 to 5, wherein the content of the lubricant is 2.0 - 6.0% (w/w) per 100% (w/w) of the formulation.

7. The oral solid formulation of any one of claims 1 to 5, wherein the content of the lubricant is 4.0 - 6.0% (w/w) per 100% (w/w) of the formulation.

8. The oral solid formulation of any one of claims 1 to 7, wherein the lubricant is sodium stearyl fumarate.

9. The oral solid formulation of any one of claims 1 to 8, which further comprises an excipient.

10. The oral solid formulation of claim 9, wherein the excipient is a hydrophilic excipient.

11. The oral solid formulation of claim 10, wherein the hydrophilic excipient is any one selected from sugar alcohol, pregelatinized starch, lactose hydrate, and microcrystalline cellulose, or a combination of two or more thereof.

12. The oral solid formulation of claim 10, wherein the hydrophilic excipient is any one selected from sugar alcohol, pregelatinized starch, and microcrystalline cellulose, or a combination of two or more thereof.

13. The oral solid formulation of claim 10, wherein the hydrophilic excipient is any one selected from sugar alcohol and pregelatinized starch, or a combination of two or more thereof.

14. The oral solid formulation of claim 10, wherein the hydrophilic excipient is a combination of sugar alcohol and pregelatinized starch.

15. The oral solid formulation of any one of claims 11 to 14, wherein the pregelatinized starch is partially pregelatinized starch.

16. The oral solid formulation of any one of claims 11 to 15, wherein the sugar alcohol is D-mannitol.

17. The oral solid formulation of any one of claims 1 to 16, which further comprises a disintegrant.

18. The oral solid formulation of claim 17, wherein the content of the disintegrant is 0.5 - 50.0% (w/w) per 100% (w/w) of the formulation.

19. The oral solid formulation of claim 17, wherein the content of the disintegrant is 0.5 - 30.0% (w/w) per 100% (w/w) of the formulation.

20. The oral solid formulation of claim 17, wherein the content of the disintegrant is 0.5 - 20.0% (w/w) per 100% (w/w) of the formulation.

21. The oral solid formulation of any one of claims 17 to 20, wherein the disintegrant is any one selected from croscarmellose sodium, sodium starch glycolate, and crospovidone, or a combination of two or more thereof.

22. The oral solid formulation of any one of claims 17 to 20, wherein the disintegrant is any one selected from croscarmellose sodium and sodium starch glycolate, or a combination of two or more thereof.

23. The oral solid formulation of any one of claims 17 to 20, wherein the disintegrant is croscarmellose sodium.

24. The oral solid formulation of any one of claims 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof is 0.5 - 70% (w/w) per 100% (w/w) of the formulation.

25. The oral solid formulation of any one of claims 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof is 10 - 50% (w/w) per 100% (w/w) of the formulation.

26. The oral solid formulation of any one of claims 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof is 15 - 40% (w/w) per 100% (w/w) of the formulation.

27. The oral solid formulation of any one of claims 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]-N,N-di(propan-2-yl)benzamide or a pharmaceutically acceptable salt thereof, or a hydrate or solvate thereof is 15 - 38% (w/w) per 100% (w/w) of the formulation.

28. The oral solid formulation of any one of claims 1 to 23, wherein the content of the 5-fluoro-2-[(4-{7-[(1S,3S,4R)-5-methylidene-2-azabicyclo[2.2.2]octane-3-carbonyl]-2,7-diazaspiro[3.5]nonan-2-yl}pyrimidin-5-yl)oxy]=-N,N-di(propan-2-yl)benzamide or a pharmaceutically acceptable salt thereof, or a hydrate or solvate thereof is 20 - 38% (w/w) per 100% (w/w) of the formulation.

29. The oral solid formulation of any one of claims 1 to 28, which is prepared by fluid-bed granulation.

30. The oral solid formulation of any one of claims 1 to 29, which is film-coated with a coating agent.

31. The oral solid formulation of any one of claims 1 to 30, which is in use for treating and/or preventing a tumor.
